(19) Europäisches Patentamt / European Patent Office / Office européen des brevets

(11) **EP 4 172 356 B1**

(12) **EUROPÄISCHE PATENTSCHRIFT**

(45) Veröffentlichungstag und Bekanntmachung des Hinweises auf die Patenterteilung:
**30.07.2025   Patentblatt 2025/31**

(21) Anmeldenummer: **21735665.8**

(22) Anmeldetag: **22.06.2021**

(51) Internationale Patentklassifikation (IPC):
**C12Q 1/6806** *(2018.01)*     **B01L 7/00** *(2006.01)*
**C12N 15/10** *(2006.01)*

(52) Gemeinsame Patentklassifikation (CPC):
(C-Sets verfügbar)
**C12N 15/1013; B01L 7/52; C12Q 1/6806;**
B01L 2200/0668; B01L 2300/0851;
B01L 2300/1827; B01L 2400/043        (Forts.)

(86) Internationale Anmeldenummer:
**PCT/EP2021/067049**

(87) Internationale Veröffentlichungsnummer:
**WO 2021/259960 (30.12.2021 Gazette 2021/52)**

(54) **VERFAHREN UND VORRICHTUNG ZUR EXTRAKTION UND AMPLIFIKATION EINER TARGET-NUKLEINSÄURE**

METHOD AND DEVICE FOR EXTRACTING AND/OR AMPLIFYING A TARGET NUCLEIC ACID

PROCÉDÉ ET DISPOSITIF D'EXTRACTION ET/OU D'AMPLIFICATION D'UN ACIDE NUCLÉIQUE CIBLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.06.2020   DE 102020116930**

(43) Veröffentlichungstag der Anmeldung:
**03.05.2023   Patentblatt 2023/18**

(73) Patentinhaber: **HP Health Solutions Germany GmbH**
**82166 Gräfelfing (DE)**

(72) Erfinder:
• **STEHR, Joachim**
**82166 Gräfelfing (DE)**
• **BUERSGENS, Federico**
**82166 Gräfelfing (DE)**
• **REBUFFO-SCHEER, Cecilia**
**82166 Gräfelfing (DE)**
• **GROTZ, Bettina**
**82166 Gräfelfing (DE)**
• **DUCZEK, Juliane**
**82166 Gräfelfing (DE)**
• **SCHMIDBAUER, Simon**
**82166 Gräfelfing (DE)**

(74) Vertreter: **Tautz & Schuhmacher**
**Nibelungenstraße 84**
**80639 München (DE)**

(56) Entgegenhaltungen:
WO-A1-2015/086652     WO-A1-89/11546
WO-A2-2008/020381     WO-A2-2012/006116

(52) Gemeinsame Patentklassifikation (CPC): (Forts.)

C-Sets
**C12Q 1/6806, C12Q 2527/101, C12Q 2563/143,
C12Q 2563/149, C12Q 2565/518, C12Q 2565/519,
C12Q 2565/537**

**Beschreibung**

Bereitgestellt werden Verfahren zur Extraktion und/oder Amplifikation einer Target-Nukleinsäure, eine Vorrichtung zur Amplifikation einer Target-Nukleinsäure und eine Verwendung von magnetischen Mikropartikeln zur Extraktion einer Target-Nukleinsäure. Die Ausführungsformen liegen somit insbesondere auf dem Gebiet der Molekulardiagnostik.

In der Molekulardiagnostik sind Verfahren bekannt, mittels welcher die Anwesenheit oder Abwesenheit von Target-Nukleinsäuren in einer zu untersuchenden Probe bestimmt werden kann. Diese Verfahren weisen jedoch eine limitierte Sensitivität auf und erfordern daher oftmals eine MindestKonzentration an vorhandener Target-Nukleinsäure, um deren Anwesenheit zuverlässig bestimmen zu können. Es ist daher oftmals erforderlich, vor einer zur Detektion durchzuführenden Amplifikationsreaktion zunächst die Target-Nukleinsäure, sofern vorhanden, aus einer Probenflüssigkeit zu extrahieren, bevor die Amplifikationsreaktion durchgeführt wird, beispielsweise mittels einer PCR.

Alternativ oder zusätzlich kann versucht werden, die Target-Nukleinsäure in jenem Bereich der Reaktionslösung zu konzentrieren, in welchem die Amplifikationsreaktion primär stattfindet. Beispielsweise offenbart die Druckschrift DE 44 09 436 A1 ein Verfahren, bei welchem die Target-Nukleinsäuren chemisch und/oder physikalisch an ein Heizelement gebunden werden, indem das Heizelement funktionalisiert wird oder die Target-Nukleinsäuren mittels magnetischer Teilchen zum Heizelement bewegt werden.

WO2008/020381A2 beschreibt magnetische Partikel, die mit Oligonukleotiden konjugiert sind und dazu dienen, eine Zielnukleinsäure magnetisch zu einem Sensor zu bewegen, um die Konzentration zu messen. WO2015/086652A1 beschreibt ein Verfahren, bei dem eine Target-DNA an funktionalisierte Magnetkügelchen bindet und magnetisch zu einer bestimmten Oberfläche bewegt wird. WO2012/006116A2 beschreibt ein Verfahren zur Vorbereitung einer Nukleinsäureprobe für die Sequenzierung. WO89/11546A1 beschreibt einen Primer, der für effizientes Waschen an magnetische Partikel funktionalisiert ist.

Es ist die Aufgabe, ein Verfahren und eine Vorrichtung bereitzustellen, welche sich für eine effiziente Extraktion und/oder Amplifikation einer Target-Nukleinsäure eignen und dadurch das Detektionslimit für den Nachweis von Nukleinsäuren verbessern.

Diese Aufgabe wird gelöst durch Verfahren, Vorrichtungen und Verwendungen mit den Merkmalen der jeweiligen unabhängigen Ansprüche. Vorteilhafte Ausgestaltungen sind in den Unteransprüchen und in der Beschreibung angegeben.

Eine Ausführungsform betrifft ein Verfahren zur Extraktion einer Target-Nukleinsäure aus einer Probenflüssigkeit. Das Verfahren umfasst ein Bereitstellen einer Probenflüssigkeit mit der Target-Nukleinsäure in einem Reaktionsgefäß, sowie ein Bereitstellen von magnetischen Mikropartikeln in der Probenflüssigkeit, die jeweils mit zumindest einer Extraktionsnukleinsäure funktionalisiert sind, wobei die Extraktionsnukleinsäuren zumindest teilweise komplementär zur Target-Nukleinsäure sind. Zudem umfasst das Verfahren ein Hybridisieren zumindest eines Teils der Target-Nukleinsäure mit einer der Extraktionsnukleinsäuren und ein Binden der Target-Nukleinsäure über die Extraktionsnukleinsäure an eines der magnetischen Mikropartikeln. Optional kann das Hybridisieren ein Temperieren zumindest eines Teils der Probenflüssigkeit derart umfassen, dass die Target-Nukleinsäure mit einer der Extraktionsnukleinsäuren hybridisiert und über die Extraktionsnukleinsäure an eines der magnetischen Mikropartikeln bindet. Ferner umfasst das Verfahren ein Bereitstellen eines Magnetfeldes in dem Reaktionsgefäß derart, dass sich zumindest ein Teil der mit der Target-Nukleinsäure verbundenen magnetischen Mikropartikeln an einem in und/oder an dem Reaktionsgefäß angeordneten Extraktionselement anlagert.

Eine weitere Ausführungsform betrifft ein Verfahren zur Amplifikation einer Target-Nukleinsäure. Das Verfahren umfasst ein Bereitstellen von magnetischen Mikropartikeln in einer Reaktionslösung, welche jeweils mit zumindest einem Primer funktionalisiert sind und über den zumindest einen Primer mit zumindest einer Target-Nukleinsäure verbindbar oder verbunden sind, sowie ein Bereitstellen eines Lokalheizelements in direktem Kontakt mit der Reaktionslösung. Zudem umfasst das Verfahren ein Aussetzen zumindest eines Teils der mit der Target-Nukleinsäure verbundenen magnetischen Mikropartikeln in der Reaktionslösung einem Magnetfeld derart, dass sich zumindest ein Teil der magnetischen Mikropartikel an dem Lokalheizelementanlagern, sowie ein lokales Erhitzen der Reaktionslösung auf eine Denaturierungstemperatur mittels des Lokalheizelements in dem Bereich, in welchem die magnetischen Mikropartikel an das Lokalheizelement angelagert sind.

Eine weitere Ausführungsform betrifft ein Verfahren zur Amplifikation einer Target-Nukleinsäure. Das Verfahren umfasst dabei die folgenden Schritte:

a) Bereitstellen einer die Target-Nukleinsäure (12) beinhaltenden Probenflüssigkeit in einem Reaktionsgefäß und zumindest eines Lokalheizelementes in direktem Kontakt mit der Probenflüssigkeit;
b) Bereitstellen magnetischer Mikropartikel in der Probenflüssigkeit, wobei die magnetischen Mikropartikel jeweils mit zumindest einem Primer für die Amplifikation der Target-Nukleinsäure funktionalisiert sind;
c) Hybridisieren der Target-Nukleinsäure mit zumindest einem der auf den magnetischen Mikropartikeln funktionalisierten Primern;
d) Bereitstellen eines Magnetfelds im Reaktionsgefäß derart, dass sich zumindest ein Teil der magnetischen Mikropartikel mit der daran hybridisierten Target-Nukleinsäure an das Lokalheizelement anlagert;

e) Entfernen der Probenflüssigkeit aus dem Reaktionsgefäß;

f) Bereitstellen einer Reaktionslösung zur Durchführung einer Amplifikationsreaktion der Target-Nukleinsäure in dem Reaktionsgefäß;

g) Lokales Erhitzen der Reaktionslösung auf eine Temperatur in einem Denaturierungstemperatur mittels des zumindest einen Lokalheizelements in dem Bereich, in welchem die magnetischen Mikropartikel an das Lokalheizelement angelagert sind.

Eine weitere Ausführungsform betrifft eine Vorrichtung zur Amplifikation einer Target-Nukleinsäure. Die Vorrichtung umfasst ein Reaktionsgefäß, welches dazu ausgelegt ist, eine die Target-Nukleinsäure enthaltende Reaktionslösung aufzunehmen, sowie zumindest ein Lokalheizelement, welches derart in und/oder an dem Reaktionsgefäß angeordnet ist, dass sich das Lokalheizelement bei mit Reaktionslösung befülltem Reaktionsgefäß zumindest teilweise in direktem Kontakt mit der Reaktionslösung befindet. Außerdem umfasst die Vorrichtung einen Magneten zur Erzeugung eines Magnetfeldes, wobei das Magnetfeld auf zumindest einen Teil der in der Reaktionslösung befindlichen magnetischen Mikropartikel derart wirkt, dass sich diese an das Lokalheizelement anlagern.

Eine weitere Ausführungsform betrifft eine Verwendung von magnetischen Mikropartikeln zur Extraktion einer Target-Nukleinsäure aus einer Probenflüssigkeit.

eine weitere Ausführungsform betrifft ein magnetisches Mikropartikel, welches mit zumindest einem Primer für eine Amplifikationsreaktion einer Target-Nukleinsäure funktionalisiert ist.

Manche Ausführungsformen bieten den Vorteil, dass bei der Extraktion der Target-Nukleinsäure aus der Probenflüssigkeit ein zuverlässiges Hybridisieren der in der Probenflüssigkeit befindlichen Target-Nukleinsäure mit den an die Mikropartikel funktionalisierten Extraktionsnukleinsäuren erzielt werden kann, da durch die in Suspension befindlichen Mikropartikel in der Probenflüssigkeit ein geringer durchschnittlicher Abstand der Mikropartikel mit den in der Probenflüssigkeit befindlichen Target-Nukleinsäuren erreicht werden kann. Insbesondere bieten manche Ausführungsformen dabei den Vorteil, dass der mittlere Abstand der magnetischen Mikropartikel in der Probenflüssigkeit und damit einhergehend auch der mittlere Abstand eines magnetischen Mikropartikels zu einer Target-Nukleinsäure durch eine geeignete Wahl der Konzentration der Mikropartikel in der Probenflüssigkeit bestimmt werden kann.

Zudem bieten manche Ausführungsformen den Vorteil, dass diese effiziente Hybridisierung mit einer effizienten Möglichkeit zur Separation der Target-Nukleinsäure von der restlichen Probenflüssigkeit kombiniert werden kann, da durch die Bereitstellung eines Magnetfeldes und entsprechender Magnetkräfte die magnetischen Mikropartikel gezielt angesprochen werden können und im Reaktionsgefäß in eine gewünschte Richtung bewegt werden können, während die restliche Probenflüssigkeit bzw. Reaktionslösung dadurch nicht oder nahezu nicht beeinflusst wird. Auf diese Weise können die magnetischen Mikropartikel und entsprechend die daran funktionalisierten und hybridisierten Target-Nukleinsäuren an einer Gefäßwand angelagert werden, während die restliche Probenflüssigkeit davon unberührt im Reaktionsgefäß verbleibt und aus diesem entfernt werden kann.

Ferner bieten manche Ausführungsformen den Vorteil, dass auch bei der Amplifikation die Target-Nukleinsäuren, welche an die funktionalisierten, magnetischen Mikropartikel hybridisiert ist, auf einfache Weise in den Bereich des lokalen Heizens bewegt werden können. Auch dabei kann dies mittels eines Magnetfelds und der dadurch auf die magnetischen Mikropartikel wirkenden Magnetkräfte erzielt werden, während die Reaktionslösung davon unberührt bleibt. Somit können die magnetischen Mikropartikel optional in mehrfacher Weise nutzbringend für den Nachweis einer Target-Nukleinsäure eingesetzt werden, nämlich zum einen für eine effiziente Extraktion der Target-Nukleinsäuren aus einer Probenflüssigkeit und zum anderen für eine effiziente Konzentration der Target-Nukleinsäuren in dem Bereich, welcher im Rahmen einer Amplifikationsreaktion lokal beheizt wird.

Magnetische Mikropartikel sind dabei Mikropartikel, welche ferromagnetische oder paramagnetische Eigenschaften aufweisen. Die Größe der Mikropartikel liegt dabei optional in einem Bereich von ca. 10 nm bis zu etwa 2 mm, optional in einem Bereich von 100 nm bis 1 mm, optional in einem Bereich von 500 nm bis 50 $\mu$m. Die Form der Mikropartikel ist dabei frei wählbar und kann beispielsweise kugelförmig, würfelförmig, quaderförmig, oder ellipsoid ausgebildet sein. Optional sind die magnetischen Mikropartikel mit ferromagnetischen Eigenschaften aus zumindest einem der folgenden Materialen ausgebildet oder beinhalten zumindest eines der folgenden Materialien: Eisen, Nickel, Cobalt, AlNiCo, SmCo, $Nd_2Fe_{14}B$, $Ni_{80}Fe_{20}$ ("Permalloy"), und/oder NiFeCo-Legierungen. Optional sind die magnetischen Mikropartikel mit paramagnetischen Eigenschaften aus zumindest einem der folgenden Materialien ausgebildet oder beinhalten zumindest eines der folgenden Materialien: Erdalkalimetalle, Alkalimetalle, und/oder seltene Erden. Alternativ kann ein magnetischer Mikropartikel aus einem nicht-magnetischen Material ausgebildet sein, wie etwa aus Glas und/oder Silikat, wobei darin magnetische Stoffe eingebettet sind. Beispielsweise kann ein solcher Mikropartikel einen Kern aus magnetischen Materialien aufweisen. Die magnetischen Mikropartikel sind dabei optional mit einer oder mehreren Beschichtungen versehen, um eine Funktionalisierung mit Nukleinsäuren, insbesondere mit Extraktionsnukleinsäuren und/oder Primern, zu ermöglichen oder zu begünstigen. Optional sind an einem Mikropartikel mindestens eine Extraktionsnukleinsäure und/oder ein Primer und maximal $10^{12}$ Extraktionsnukleinsäuren und/oder Primer funktionalisiert. Optional liegt eine Flächendichte von Extraktionsnukleinsäuren und/oder Primern, die an die Oberfläche eines magnetischen Mikropartikels funktionalisiert sind, in einem Bereich von 0.0001 bis 1 pro Quadratnanometer. Optional können die Mikropartikel eine Beschichtung aufweisen, welche eine Funktionalisierung mit Oligonukleotiden ermöglicht und/oder erleichtert. Beispielsweise können die magnetischen Mikropartikel an deren Oberfläche zumindest teilweise mit Streptavidin funktionalisiert sein.

Die Probenflüssigkeit ist optional eine Ausgangsflüssigkeit, die die Target-Nukleinsäure enthält (z.B. Probenmaterial, das die Target-Nukleinsäure enthält, oder Probenmaterial, in der die Target-Nukleinsäure (zuvor) freigesetzt wurde (z.B. aus Pathogenen und/oder Zellen) oder eine Flüssigkeit, in der die Nukleinsäure bereits im aufgereinigten Zustand vorliegt). Die Probenflüssigkeit kann optional auch Reagenzien aufweisen oder mit solchen versehen werden, die das Hybridisieren der Target-Nukleinsäure an die Funktionsnukleinsäure bzw. Extraktionsnukleinsäure bzw. das Primeroligonukleotid auf dem Lokalheizelement begünstigt, wie etwa entsprechende Salze.

Das Extraktionselement ist kann dabei einen Teil des Reaktionsgefäßes darstellen und/oder fest mit dem Reaktionsgefäß verbunden sein, oder unabhängig und separat vom Reaktionsgefäß ausgebildet sein. Optional kann beispielsweise eine Gefäßwand und/oder der Boden und/oder der Deckel des Reaktionsgefäßes ein Extraktionselement darstellen. Alternativ kann das Extraktionselement durch eine separate Vorrichtung gebildet sein, wie etwa ein oder mehrere Drähte und/oder eine Folie, welche in der Reaktionslösung angeordnet werden kann. Dabei kann beispielsweise das Extraktionselement in und/oder an dem Reaktionsgefäß befestigt werden. Optional kann das Extraktionselement zumindest teilweise ferromagnetisch ausgebildet sein, um die magnetischen Mikropartikel anzuziehen.

Dass sich die magnetischen Mikropartikel an das Extraktionselement anlagern bedeutet dabei, dass diese an einer Oberfläche des Extraktionselement angeordnet sind und nicht mehr frei in der Reaktionslösung bzw. Probenflüssigkeit suspendiert sind. Beispielsweise könne die sich an das Extraktionselement oder an das Lokalheizelement anlagernden Mikropartikel an dem Extraktionselement bzw. am Lokalheizelement sedimentieren, wobei die Sedimentation nicht zwangsläufig ausschließlich oder primär durch die Einwirkung der Schwerkraft verursacht wird, sondern primär durch das bereitgestellte Magnetfeld verursacht wird. Die angelagerten Mikropartikel sind dabei optional derart fest mit dem Extraktionselement bzw. dem Lokalheizelement verbunden, dass diese auch bei einem Entfernen der Reaktionslösung und/oder der Probenflüssigkeit zumindest teilweise am Extraktionselement bzw. Lokalheizelement angelagert bleiben.

Optional kann eine Gefäßwand als Extraktionselement ausgebildet sein. Die Gefäßwand des Reaktionsgefäßes, an welche sich die Mikropartikel bei der Extraktion der Target-Nukleinsäure anlagern, kann dabei von jener Gefäßwand des Reaktionsgefäßes abweichen, an welcher sich die Mikropartikel bei der Amplifikation der Target-Nukleinsäure anlagern. Optional lagern sich die Mikropartikel bei der Extraktion der Target-Nukleinsäure nicht an jener Gefäßwand an, welche das Lokalheizelement aufweist oder als das Lokalheizelement ausgebildet ist. Bei der Amplifikation der Target-Nukleinsäure ist jedoch eine Anlagerung der magnetischen Mikropartikel an jener Gefäßwand vorgesehen, welche das Lokalheizelement aufweist oder als dieses ausgebildet ist. Das optionale Anlagern an unterschiedlichen Gefäßwänden bei der Extraktion einerseits und der Amplifikation andererseits bietet den Vorteil, dass dadurch das Entstehen unerwünschter Ablagerungen am Lokalheizelement während der Extraktion der Target-Nukleinsäure aus der Probenflüssigkeit vermieden werden kann, welche ansonsten zu einer Beeinträchtigung des lokalen Heizens und/oder der Durchführung der PCR führen könnten. Gemäß einer Optionalen Ausführungform ist jedoch das Anlagern der extrahierten Mikropartikel direkt an dem Lokalheizelement für die durchzuführende Amplifikationsreaktionsreaktion vorteilhaft.

Die Probenflüssigkeit ist optional eine Flüssigkeit, welche gegebenenfalls die Target-Nukleinsäure aufweist und auch noch andere Bestandteile umfasst. Insbesondere kann die Probenflüssigkeit noch weitere Bestandteile aufweisen, welche nicht mit extrahiert werden sollen. Diese anderen Bestandteile können andere Nukleinsäuren umfassen, also Nukleinsäuren, welche eine andere Nukleotidsequenz aufweisen. Auch können die anderen Bestandteile Verunreinigungen aufweisen, welche von nicht-nukleotidischer Art sind. Insbesondere kann es sich bei der Probenflüssigkeit um eine Flüssigkeit handeln, welche beispielsweise menschlichen und/oder tierischen und/oder pflanzlichen und/oder anderweitigen organischen Ursprungs ist. Beispielsweise kann die Probenflüssigkeit Blut und/oder Sekret und/oder Körperausscheidungen und/oder Absonderungen von Schleimhäuten und/oder Speichel und/oder Zellflüssigkeit enthalten oder daraus bestehen. Die Probenflüssigkeit kann optional vor dem Extrahieren einer oder mehreren Behandlungen unterzogen worden sein, um etwa in der Probenflüssigkeit enthaltene Nukleinsäuren zumindest teilweise freizusetzen. Beispielsweise kann die Probenflüssigkeit einer Behandlung unterzogen worden sein, um etwa darin befindliche Zellen zu lysieren bzw. aufzuschließen, um die ggf. darin befindlichen Nukleinsäuren zumindest teilweise aus den Zellen herauszulösen, sodass die Nukleinsäuren optional frei in der Probenflüssigkeit vorliegen und zumindest teilweise nicht oder nicht mehr in Zellkernen und/oder Zellen eingeschlossen sind. Das Lysieren erfolgt optional derart, dass die freigesetzten Nukleinsäuren zumindest nicht vollständig zerstört werden und besonders optional vollständig erhalten bzw. unversehrt bleiben. Insbesondere kann die Probenflüssigkeit optional derart vorliegen, dass ein Durchführen einer Amplifikationsreaktion zur Vervielfältigung der extrahierten Nukleinsäure in der Probenflüssigkeit nicht möglich ist. So kann die Probenflüssigkeit beispielsweise physikalische und/oder chemische und/oder biologische Eigenschaften aufweisen, welche einer Durchführung einer Amplifikationsreaktion, wie beispielsweise einer PCR, in der Probenflüssigkeit entgegenstehen. Beispielsweise kann die Probenflüssigkeit eine Viskosität und/oder einen pH-Wert und/oder eine Salzkonzentration und/oder Polarität und/oder Enzyme und/oder Proteasen aufweisen, welche die Durchführung einer Amplifikationsreaktion nicht ermöglichen, beispielsweise weil die Aktivität der hierzu notwendigen Polymerase-Enzyme inhibiert wird oder Enzyme wie Proteasen vorliegen, die die Polymerase-Enzyme abbauen können.

Die Probenflüssigkeit ist dabei derart beschaffen, dass die magnetischen Mikropartikel in der Probenflüssigkeit suspendiert werden können und ein Hybridisieren von einzelsträngig vorliegender Target-Nukleinsäure an die an die magnetischen Mikropartikel funktionalisierten Primer bzw. Extraktionsnukleinsäuren ermöglicht wird.

Optional kann zumindest ein Parameter der Probenflüssigkeit angepasst werden, um ein Hybridisieren der Target-Nukleinsäure an die Funktionsnukleinsäure angesichts des vorliegenden Maßes an Komplementarität zu ermöglichen. Beispielsweise kann eine Konzentration von $MgCl_2$ in der Probenflüssigkeit erhöht werden, um auch ein Hybridisieren bei geringer Komplementarität zu ermöglichen, wohingegen optional die Konzentration von $MgCl_2$ in der Probenflüssigkeit verringert werden kann, um ein Hybridisieren erst ab einem bestimmten höheren Maß an Komplementarität zu ermöglichen.

Die Reaktionslösung weist optional Reagenzien auf, die zur Vervielfältigung mittels Polymerase Kettenreaktion nötig sind und kann mit dem Lokalheizelement und den magnetischen Mikropartikeln in Kontakt gebracht werden, sodass die magnetischen Mikropartikeln in der Reaktionslösung suspendiert werden.

Die Reaktionslösung ist dabei optional eine Flüssigkeit, in welcher die Target-Nukleinsäure als solche einzelsträngig und/oder doppelsträngig vorliegend überdauern kann und/oder stabilisiert wird. Jedoch unterscheidet sich die Reaktionslösung von der Probenflüssigkeit. Mit anderen Worten ist die Reaktionslösung optional derart ausgestaltet, dass die Target-Nukleinsäure durch die Wechselwirkung mit der Reaktionslösung keinen Schaden nimmt. Beispielsweise kann die Reaktionslösung als eine wässrige Lösung und/oder als eine Pufferlösung vorliegen. Sofern nach dem Extrahieren der Target-Nukleinsäure eine Amplifikation der Target-Nukleinsäure beabsichtigt ist, kann die Reaktionslösung optional derart ausgestaltet sein, dass die Reaktionslösung die Durchführung einer solchen Amplifikationslösung ermöglicht. Beispielsweise kann die Reaktionslösung als eine Pufferlösung ausgebildet sein, in welcher die Durchführung einer PCR möglich ist, um die Target-Nukleinsäure zumindest teilweise zu vervielfältigen. Die Reaktionslösung wird optional in einem Reaktionsgefäß bereitgestellt. Die bereitgestellte Reaktionslösung weist optional ein Volumen von zumindest 1 μl und nicht mehr als 10 ml, mehr optional von zumindest 5 μl und nicht mehr als 1 ml, am meisten optional von zumindest 10 μl und nicht mehr als 100 μl auf.

Die Bezeichnungen "Nukleinsäure" und "Oligonukleotid" umfassen im Zusammenhang mit der vorliegenden Offenbarung nicht nur (Desoxy)-Ribonukleinsäuren bzw. (Desoxy)-Oligo-Ribonukleotide, auch wenn vorgenannte optional sind, sondern auch Nukleinsäuren und Oligonukleotide, die ein oder mehrere Nukleotid-Analoga mit Modifikationen an ihrem Backbone (zum Beispiel Methylphosphonate, Phosphothioate oder Peptide Nucleic Acids (PNA), insbesondere an einem Zucker des Backbone (zum Beispiel 2'-O-Alkylderivate, 3'- und/oder 5'-Aminoribosen, Locked Nucleic Acids [LNA], Hexitol Nucleic Acids, Morpholinos, Glycol Nucleic Acid (GNA), Threose Nucleic Acid (TNA) oder Tricyclo-DNA, vergleiche hierzu den Aufsatz von D. Renneberg und C.J. Leumann, "Watson-Crick base-pairing properties of Tricyclo-DNA", J. Am. Chem. Soc., 2002, Bd. 124, Seiten 5993-6002, dessen diesbezüglicher Inhalt durch Verweis Teil der vorliegenden Offenbarung ist) enthalten oder die Basen-Analoga enthalten, zum Beispiel 7-Deazapurine oder Universalbasen wie Nitroindol oder modifizierte natürliche Basen wie N4-Ethyl-Cytosin. In einer Ausführung sind die Nukleinsäuren oder Oligonukleotide Konjugate oder Chimären mit nichtnukleosidischen Analoga, zum Beispiel PNA. Die Nukleinsäuren oder Oligonukleotide enthalten in einer Ausführungsform an einer oder mehreren Positionen nicht-nukleosidische und/oder nicht-nukleotidische Einheiten wie Spacer, zum Beispiel Hexaethylenglycol oder Cn-Spacer mit n zwischen 3 und 6. Soweit die Nukleinsäuren oder Oligonukleotide Modifikationen enthalten, sind diese so gewählt, dass auch mit der Modifikation eine Hybridisierung mit natürlichen DNA/RNA-Analyten möglich ist. Optionale Modifikationen beeinflussen das Schmelzverhalten, optional die Schmelztemperatur, insbesondere um Hybride mit unterschiedlichen Graden der Komplementarität ihrer Basen unterscheiden zu können (Mismatch-Diskriminierung). Optionale Modifikationen umfassen LNA, 8-Aza-7-Deaza- Purine, 5-Propinyl-Uracil und -Cytosin und/oder abasische Unterbrechungen oder Modifikationen in der Nukleinsäure oder in dem Oligonukleotid. Weitere Modifikationen im Sinne der Offenbarung sind zum Beispiel Modifikationen mit Biotin und/oder Thiol und/oder Schwefel und/oder Fluoreszenzdonor- und Fluoreszenzakzeptormolekülen und/oder Quencher.

Die Begriffe Target-Nukleinsäure und Nukleinsäure werden im Rahmen dieses Dokuments als Äquivalente verwendet, sofern nicht anders angegeben. Dass die Nukleinsäure aus der Probenflüssigkeit extrahiert wird, bedeutet dabei, dass die Nukleinsäure zumindest teilweise in und/oder von der Probenflüssigkeit isoliert wird und optional von der Probenflüssigkeit getrennt werden kann. Die aus der Probenflüssigkeit zu extrahierende Nukleinsäure kann auch als Target-Nukleinsäure oder Target-Nukleinsäure bezeichnet werden. Insbesondere kann somit das Extrahieren einer Nukleinsäure ein Separieren der Nukleinsäure von der Probenflüssigkeit umfassen oder einem solchen Separieren dienen. Dabei kann das Extrahieren der Nukleinsäure derart ausgestaltet sein, dass lediglich die zu extrahierende Nukleinsäure von der Probenflüssigkeit extrahiert wird oder auch noch andere Bestandteile der Probenflüssigkeit mit extrahiert werden, wie beispielsweise andere Nukleinsäuren. Besonders optional wird jedoch nur die zu extrahierende Nukleinsäure aus der Probenflüssigkeit extrahiert, sodass insbesondere andere Nukleinsäuren und andere Bestandteile der Probenflüssigkeit nicht mit extrahiert werden, sondern in der Probenflüssigkeit verbleiben. Optional ist nach dem Extrahieren der Nukleinsäure die Konzentration und/oder die Anzahl von Exemplaren der extrahierten Nukleinsäure in der Probenflüssigkeit geringer, als vor dem Extrahieren, sofern überhaupt vor dem Extrahieren die zu extrahierende Nukleinsäure in der Probenflüssigkeit vorhanden war.

Ein höheres Maß an Komplementarität zwischen der Extraktionsnukleinsäure und der Target-Nukleinsäure kann Vorteile hinsichtlich der Selektivität bzw. Spezifität bei der Extraktion bieten. So kann ein hohes Maß an Komplementarität zwischen der Extraktionsnukleinsäure und der die Target-Nukleinsäure ermöglichen, dass im Wesentlichen nur die Target-Nukleinsäure an die Extraktionsnukleinsäure binden kann, während ein Binden anderer Nukleinsäuren aus der Probenflüssigkeit an die Funktionsnukleinsäure eine Ausnahme darstellt. Andererseits kann ein geringes Maß an Komplementarität zwischen der Extraktionsnukleinsäure und der Target-Nukleinsäure optional ein Binden von unterschiedlichen Nukleinsäuren aus der Probenflüssigkeit an die Extraktionsnukleinsäure ermöglichen, so dass auch andere Nukleinsäuren aus der Probenflüssigkeit extrahiert werden können und/oder die Target-Nukleinsäure auch dann extrahiert werden kann, wenn die Extraktionsnukleinsäure ein nur geringes Maß an Komplementarität zu dieser aufweist, beispielsweise weil die Basensequenz der Target-Nukleinsäure nicht in ausreichendem Maß bekannt ist und entsprechend die Extraktionsnukleinsäure nicht exakt auf die Target-Nukleinsäure angepasst werden kann.

Dass ein magentischer Mikropartikel mit einer Extraktionsnukleinsäure und/oder einem Primer funktionalisiert ist bedeutet dabei, dass die Extraktionsnukleinsäure bzw. der Primer an das Mikropartikel gebunden ist. Dies wiederum bedeutet, dass die Extraktionsnukleinsäure bzw. der Primer mechanisch fest mit dem Mikropartikel verbunden ist, insbesondere durch eine chemische und/oder elektrostatische Bindung. Beispielsweise kann die Extraktionsnukleinsäure bzw. der Primer mittels einer oder mehrerer Thiol-Bindungen und/oder Schwefelbindungen an eine Oberfläche des Mikropartikel gebunden sein. Optional ist dazu das Mikropartikel an seiner Oberfläche zumindest teilweise mit einem Material versehen, das die Anbindung von Nukleinsäuren erlaubt. Zum Beispiel kann eine vergoldete Oberfläche genutzt werden, um die Extraktionsnukleinsäure und optional andere Nukleinsäuren über eine oder mehrere Thiol- und/oder Schwefel-Bindung(en) an das Lokalheizelement zu binden. Auch kann zum Beispiel eine Streptavidin-Biotin-Bindung zur Anbindung der Extraktionsnukleinsäure bzw. des Primers und/oder anderer Nukleinsäuren auf das Mikropartikel genutzt werden, wenn zum Beispiel, optional vorher, einer der beiden Partner (Streptavidin oder Biotin) auf das Mikropartikel gebunden wurde und die Funktionsnukleinsäure (optional am 5'-Ende) mit dem anderen der beiden Partner modifiziert ist und anschließend darüber an das Mikropartikel gebunden wird. Auch andere Modifikationen wie zum Beispiel Amino- oder Carboxy-Gruppen können zur Bindung der Extraktionsnukleinsäure bzw. des Primers auf das Mikropartikel genutzt werden, die Oberfläche des Lokalheizelements kann hierfür zum Beispiel, optional vorher, mit Epoxy und/oder einem Metall modifiziert werden. Optional findet eine Bindung derart statt, dass das 5'-Ende der Funktionsnukleinsäure an das Lokalheizelement gebunden ist, sodass das 3'-Ende frei ist. Dies kann insbesondere dann vorteilhaft sein, wenn die Extraktionsnukleinsäure selbst als Primer ausgebildet ist und in einer nach der Extraktion durchzuführenden Amplifikationsreaktion als Primer dienen soll.

Dass das Lokalheizelement mit der Probenflüssigkeit bzw. mit der Reaktionslösung in Kontakt ist, kann umfassen, dass eine Heizfläche des Lokalheizelements, wie etwa eine Metallfolie, in direktem Kontakt mit der Reaktionslösung bzw. der Probenflüssigkeit ist. Alternativ können eine oder mehrere Schutzschichten zwischen der Heizfläche des Lokalheizelements und der Reaktionslösung bzw. der Probenflüssigkeit angeordnet sein, wobei die eine oder die mehreren Schutzschichten eine sehr hohe Wärmeleitfähigkeit aufweisen und optional möglichst dünn ausgebildet sind.

Ein Lokalheizelement ist dabei ein Heizelement, welches sich für das lokale Heizen eines Bereichs in direkter Umgebung des Lokalheizelements eignet. Das lokale Heizen im Sinne der Offenbarung wird weiter unten im Detail erläutert.

Eine Polymerase-Kettenreaktion bzw. PCR im Sinne der vorliegenden Offenbarung ist ein Verfahren zum Vervielfältigen von Target-Nukleinsäuren, bei dem ein Vervielfältigungszyklus bestehend aus den Schritten Denaturierung, Hybridisierung und Elongation wiederholt durchlaufen wird, und zwar optional in dieser Reihenfolge. In jedem Durchlauf kann die Anzahl der Nukleinsäuremoleküle und insbesondere der Target-Nukleinsäuren vergrößert (im typischerweise besten Fall verdoppelt) werden, sodass es zu einer exponentiellen Zunahme der Zahl von Nukleinsäuremolekülen kommen kann. Im Folgenden wird eine zu vervielfältigende Target-Nukleinsäure als "Original" bezeichnet. Das Original ist ein Einzelstrang und kann zusammen mit seinem komplementären Strang, der als "Komplement" bezeichnet wird, einen Doppelstrang bilden. Das Original und auch das Komplement können Teil einer größeren Nukleinsäure sein. Insbesondere kann bei einer PCR eine in einem Durchlauf des Vervielfältigungszyklus entstandene Kopie des Originals eine Vorlage zum Bilden eines Komplements in einem folgenden Durchlauf und eine entstandene Kopie des Komplements eine Vorlage zur Bildung eines Originals in einem folgenden Durchlauf sein. Eine geläufige Bezeichnung für das Amplifikationsprodukt ist "Amplikon".

Der Denaturierungsschritt dient dazu, einen Nukleinsäure-Doppelstrang zu denaturieren, das heißt, ihn in seine beiden Einzelstränge aufzutrennen. So kann in dem Denaturierungsschritt zum Beispiel das Original von dem Komplement getrennt werden. Eine optionale Art der Denaturierung ist eine thermische Denaturierung (auch als "Schmelzen" bezeichnet). Dazu wird zumindest ein Teil des Nukleinsäure-Doppelstrangs oder der ganze Doppelstrang einer Temperatur, als "Denaturierungstemperatur" bezeichnet, ausgesetzt, die ein Trennen der Nukleinsäure-Doppelstränge hervorruft oder zumindest begünstigt. Die optionale Denaturierungstemperatur ist einerseits so hoch gewählt, dass Nukleinsäure-Doppelstränge aufgetrennt werden können. Andererseits ist die optionale Denaturierungstemperatur so niedrig gewählt, dass eine DNA-Polymerase, die sich möglicherweise ebenfalls in der Probe befindet, nicht wesentlich geschädigt wird. Ein typischer Wert für die Denaturierungstemperatur ist 95° C.

Zur Erleichterung der nachfolgenden Erläuterung der Offenbarung bezeichnet "Denaturierungsschritt" in der Nomenklatur der vorliegenden Offenbarung den Schritt des Verfahrens, in dem das Lokalheizelement Wärme erzeugt, um das Reaktionsvolumen in der direkten Umgebung des Lokalheizelements zu erwärmen und dadurch eine Denaturierung doppelsträngiger Nukleinsäuremoleküle zu bewirken. Die Dauer des Denaturierungsschritts ist demnach die Summe der Zeit, in der das Lokalheizelement in dem den Denaturierungsschritt betreffenden Durchlauf des Zyklus der PCR Wärme erzeugt. Im Fall eines Heizwiderstands und/oder eines induktiven Heizelements als Heizeinrichtung und/oder als Lokalheizelement ist die Dauer des Denaturierungsschritts also die Dauer einer Stromdurchleitung durch die Heizeinrichtung bzw. durch das Lokalheizelement, um das Reaktionsvolumen zu erwärmen und dadurch eine Denaturierung doppelsträngiger Nukleinsäuremoleküle zu bewirken. Wenn die Heizeinrichtung bzw. das Lokalheizelement in einem Durchlauf des Vervielfältigungszyklus die Wärme statt in einem in mehreren voneinander getrennten Zeitintervallen erzeugt, ist die Dauer des Denaturierungsschritts die Summe der Dauern dieser Intervalle. In dem so definierten Denaturierungsschritt ist insbesondere nicht eine Abgabe von Wärme aufgrund der des Lokalheizelements eigenen Wärmekapazität eingeschlossen, und auch nicht das Abklingen der Temperatur in dem an das Lokalheizelement angrenzenden Teil des Reaktionsvolumens, selbst wenn die dort vorhandenen Temperaturen noch innerhalb des für eine Denaturierung erforderlichen Bereichs liegen. Dies bedeutet insbesondere, dass in dem Verfahren gemäß einer Ausführungsform auch nach dem so definierten Denaturierungsschritt noch Denaturierung stattfinden kann. Es bedeutet auch, dass die im Denaturierungsschritt an das Reaktionsvolumen abgegebene Wärme in der Regel geringer ist als die im Denaturierungsschritt erzeugte Wärme. Da aber die Wärmekapazität der Lokalheizeinrichtung in einer besonders optionalen Ausführungsform vernachlässigbar ist, entspricht die Heizdauer optional der Dauer des Denaturierungsschritts.

Das Beheizen des bzw. der Lokalheizelemente, die optional als resistive Lokalheizelemente und besonders optional als Metallfolien ausgebildet sind, kann optional mittels kurzer elektrischer Impulse, mit welchen das bzw. die Lokalheizelemente bestromt werden, erreicht werden. Besonders optional erfolgt dies derart, dass nur die unmittelbare Umgebung des oder der Lokalheizelemente für kurze Zeit lokal erwärmt wird, optional, um die Denaturierung der Nukleinsäuremoleküle in dem Reaktionsvolumen durchzuführen, während der Großteil des Reaktionsvolumens, d.h. der Reaktionslösung, auf einer (in diesem Sinne "globalen") Basistemperatur verbleibt, bei der insbesondere eine Elongation, optional auch eine Hybridisierung, stattfinden kann. Dies wird optional dadurch erreicht, dass die Dauer der Erwärmung durch die Heizeinrichtung so kurz ist, dass sich das in dem umgebenden Reaktionsvolumen entstehende Wärmefeld nur wenige Mikrometer ausbreiten kann und auf diese Weise eine Aufheizzone schafft, die optional nur einen winzigen Bruchteil des Reaktionsvolumens umfasst. Insbesondere kann dadurch die eingebrachte Wärmemenge so gering sein, dass keine substantielle globale Erwärmung des Reaktionsvolumens stattfindet.

Die "globale Temperatur" im Sinne der vorliegenden Offenbarung ist die auf den Rauminhalt bezogen durchschnittliche Temperatur des Reaktionsvolumens bzw. die Reaktionslösung, in der die PCR stattfindet, also die Temperatur, die sich nach einer Thermalisierung des Reaktionsvolumens in diesem einstellt oder einstellen würde. Die "globale Erwärmung" ist die Zunahme der so definierten globalen Temperatur.

Weiter ist mittels lokalem Heizen erreichbar, dass sich nach dem Erwärmen, insbesondere im Denaturierungsschritt, die eingebrachte Wärme, die sich aus der Aufheizzone in den Rest des Reaktionsvolumens ausbreitet, dort nur eine vernachlässigbare globale Temperaturerhöhung hervorruft. "Vernachlässigbar" bedeutet hier insbesondere, dass die Temperaturerhöhung optional zu gering für eine Denaturierung der Nukleinsäuremoleküle ist und besonders optional, dass die Temperaturerhöhung zu gering ist, um die Hybridisierung und die Elongation zu stören.

Im Folgenden soll das lokale Heizen im Sinne der Offenbarung noch näher erläutert werden. Durch einen Stromfluss durch ein Lokalheizelement, welches beispielsweise als Metallfolie mit einer Dicke von etwa 20 μm oder weniger, ausgestaltet sein kann, fängt mit Beginn des Heizpulses das Lokalheizelement an, sich zu erwärmen. Das Lolkalheizelement ist optional so ausgestaltet, dass es eine möglichst große mit der Reaktionslösung in Kontakt stehenden Oberfläche und gleichzeitig eine möglichst kleine Wärmekapazität hat.

Zur Realisierung einer möglichst geringen Wärmekapazität ist das Lokalheizelement optional so ausgestaltet, dass es in mindestens einer Dimension eine Stärke von weniger als 100 μm, optional weniger als 50 μm und besonders optional weniger als 30μ m besitzt. Eine solche Begrenzung in einer Dimension könnte z.B. eine Folie sein, falls die Begrenzung in zwei Dimensionen erfolgt, kann das Lokalheizelement beispielsweise ein Draht sein und bei einer begrenzung in drei Dimensionen beispielsweise eine Kugel.

Um das Lokalheizelement nicht zu fragil zu machen, ist es zweckmäßig dass die Materialstärke in jeder Dimension mindestens 100 nm, optional 1 μm und optional 5 μm bzw. 10 μm beträgt.

Besonders optional ist die Heizvorrichtung so ausgestaltet, dass das Material eine magnetische Permeabilität von optional größer 1, optional größer 1,1 , optional größer 2, optional größer 5, optional größer 10, optional größer 20, besonders optional größer 50, optional größer 100 aufweist, so dass sich bei gegebener magnetischer Feldstärke (z.B. durch einen externen Magneten) eine hohe magnetische Flussdichte an der Oberfläche des Lokalheizelements ergibt, die zum Anziehen der magnetischen Partikel genutzt werden kannn. Dies ermöglicht zum Einen die Verwendung eines relativ schwachen Magnetfeldes zur Erreichung einer hohen magnetischen Flussdichte und zudem eine zielgerichtete Kraftwirkung auf die magnetischen Mikropartikel hin zum Lokalheizelement.

Optional ist das Lokalheizelement aus einem metallischen Material ausgebildet. Optional ist das Lokalheizelement aus ferromagnetischen Materialien wie Stahl und/oder Edelstähle und/oder Nickel und/oder sehr leitfähigen Buntmetallen ausgebildet, wie etwa Messing und/oder Kupfer. Alternativ oder zusätzlich ist das Lokalheizelement zumindest teilweise aus sehr harten Materialien ausgebildet, wie etwa Wolfram, die sehr dünne Ausgestaltungen des Lokalheizelements zulassen. Außerdem weist das Lokalheizelement optional eine sehr hohe Wärmeleitfähigkeit auf. Optional ist das Lokalheizelement derart ausgestaltet, dass es näherungsweise homogen über die Dauer des Heizpulses erwärmt. An der Oberfläche des Lokalheizelements, das während der PCR in Kontakt mit der Reaktionslösung ist, wird die Wärme vom Lokalheizelement an die Reaktionslösung übertragen, wo sie sich auf ein zunehmend größeres Volumen ausbreitet. Die Ausbreitung eines Wärmefeldes erfolgt in der Reaktionslösung durch Wärmediffusion, für die das folgende wurzelförmige Weg-Zeit-Gesetz gilt:

$$d \approx \sqrt{D \cdot t}$$

Dabei steht d für die Wegstrecke, die eine Wärmefront nach einer Zeit t entlang einer Raumrichtung in einer Reaktionslösung mit Temperaturleitfähigkeit D zurücklegt. Diese Wegstrecke d wird im Folgenden als Wärmediffusionsweite bezeichnet. Das heißt, für eine optionale typische Heizdauer von beispielsweise 100μs kann die im Lokalheizelement erzeugte Wärme in der Reaktionslösung mit einer typischen Temperaturdiffusivität (auch bekannt als Temperaturleitfähigkeit) von $D \approx 1.6 \cdot 10^{-7} m^2/s$ größenodnungsmäßig

$$d \approx \sqrt{1.6 \cdot 10^{-7} m^2/s \cdot 10^{-4} s} \approx 4\mu m$$

weit diffundieren. Mit anderen Worten hat sich in diesem Zeitraum von 100μs die Wärme, die im Lokalheizelement durch resistives Heizen erzeugt wird, in die das Lokalheizelement umgebende Reaktionslösung in etwa 4 μm weit ausgebreitet.

Der Teil des Reaktionsvolumens bzw. der Reaktionslösung, in den die Wärme während des Heizpulses diffundieren kann, wird im Folgenden als "Aufheizzone" (AHZ) bezeichnet. Die Ausdehnung der Aufheizzone senkrecht zur Oberfläche des Lokalheizelements kann näherungsweise durch die oben definierte Wärmediffusionsweite abgeschätzt werden.

Optional erfolgt das lokale Erhitzen der Reaktionslösung auf die Denaturierungstemperatur mittels des Lokalheizelements derart, dass eine Wärmediffusionsweite in die Reaktionslösung senkrecht zur Oberfläche des Lokalheizelements in einem Bereich von 0,05 μm bis 200 μm liegt.

Optional werden durch die geeignete Wahl der Heizdauer lediglich ein oder mehrere Teilvolumina der Reaktionslösung signifikant aufgeheizt, welche optional Ausdehnungen (senkrecht von der Oberfläche des Mikroheizers gemessen), d.h. Wärmediffusionsweite von optional 0,05 μm bis 200 μm, optional 0,1μm bis 100 μm, optional 0,1 μm bis 50 μm, optional 0.1 μm bis 25 μm, optional 0.1 μm bis 15 μm und optional 0,1 μm bis 10 μm aufweisen. Der Ausdruck "signifikant erwärmt werden" bedeutet dabei eine deutliche Erwärmung, wobei jedoch die Temperaturerhöhung in der Entfernung einer Wärmediffusionsweite senkrecht von der Oberfläche des Lokalheizelements optional weniger als 50 K beträgt, optional weniger als 30 K , optional weniger als 20 K, optional weniger als 10 K, optional weniger als 5 K beträgt.

Optional erfolgt das lokale Erhitzen der Reaktionslösung auf die Denaturierungstemperatur mittels des Lokalheizelements derart, dass eine Temperaturerhöhung der Reaktionslösung in einem Abstand vom Lokalheizelement, der das Doppelte der Wärmediffusionsweite beträgt, durch das Beheizen des Lokalheizelements nicht mehr als 5 K beträgt. Einerseits soll durch eine ausreichende räumliche Ausdehnung des erwärmten Bereichs der Reaktionslösung senkrecht zur Oberfläche des Lokalheizelements erreicht werden, dass die auf dem Lokalheizelement entstehenden Amplikons, die typischerweise eine Länge von 0,02 - 3 $\mu$m haben (entsprechend ca. 60-10000 Basenpaaren), möglichst homogen beheizt und somit denaturiert werden können. Zum Anderen soll die Wärmediffusionsweite optional ausreichend klein ausgebildet sein, um das Volumenverhältnis von Aufheizzone zum unbeheizten Passiv-Volumen der Reaktionslösung gering zu halten.

Eine Lokalisierung des Wärmefeldes lässt sich erreichen, indem die Heizdauer bzw. die Dauer des Denaturierungsschritts optional kleiner als 20 ms, optional kleiner als 10 ms, optional kleiner als 5 ms, optional kleiner als 3 ms, optional kleiner als 2 ms, optional kleiner als 1 ms gewählt wird. Optional beträgt demnach eine Heizdauer des Lokalheizelements zum lokalen Erhitzen der Reaktionslösung auf die Denaturierungstemperatur nicht mehr als 20 ms pro Denaturierungsschritt.

Optional lässt sich durch die Wahl geeigneter Heizdauern die räumliche Ausbreitung des Temperaturfeldes steuern. Dies gilt in einem Nicht-Gleichgewichtszustand, d.h. solange sich kein stationärer Wärmegradient ausgebildet hat, der im stationären Zustand lediglich von der Geometrie der Heizeinrichtung und den Randbedingungen abhängt. Entsprechend erfolgt das lokale Erhitzen der Reaktionslösung auf die Denaturierungstemperatur mittels des Lokalheizelements optional derart, dass kein stationärer Wärmegradient in der Reaktionslösung erzeugt wird.

Durch die räumliche Ausbreitung der Wärme entsprechend obiger Gleichung verteilt sich die vom Lokalheizelement in die Reaktionslösung eingebrachte Wärmemenge auf ein zunehmend größeres Volumen der Reaktionslösung, so dass sich senkrecht zur Oberfläche des Lokalheizelement, das um eine Temperatur $\Delta T_{Local}$ (oder auch $\Delta T$ bezeichnet) heißer ist, als die globale Durchschnittstemperatur, ein mittlerer Temperaturgradient von $\Delta T/d$ ($\Delta T/\sqrt{D \cdot t}$, wobei t hier für die Dauer des Heizschritts steht) ergibt, der den Wärmetransport ermöglicht.

Dadurch können beispielsweise Temperaturgradienten erreicht werden, die optional größer als 1K/$\mu$m, optional größer als 3K/$\mu$m und ganz besonders optional größer als 5K/$\mu$m erreichen, um eine hohe Lokalisierung der Temperaturerhöhung zu erreichen. Alternativ oder zusätzlich sind die Wärmegradienten optional kleiner als 1000K/$\mu$m und besonders optional kleiner als 300K/$\mu$m. Dies kann vorteilhaft sein, um thermophoretische Effekte in der Reaktionslösung zu vermeiden.

Eine genauere Abschätzung der räumlichen Wärmeausbreitung während und nach dem Heizpuls für eine jeweilige Geometrie des Lokalheizelements kann beispielsweise durch Finite-Elemente Methoden, wie z.B. mit kommerziellen Lösungen wie COMSOL erreicht werden, die ein numerisches Lösen der Wärmediffusionsgleichung ermöglichen.

Optional wird die PCR derart durchgeführt, dass in mindestens einem der Durchläufe des Vervielfältigungszyklus der PCR die Heizeinrichtung bzw. das Lokalheizelement bzw. die Lokalheizelemente dem Reaktionsvolumen bzw. der Reaktionslösung weniger im Denaturierungsschritt erzeugte Wärme zuführen, als CR * 5° C, und wobei CR die Wärmekapazität des Reaktionsvolumens während des Erwärmens durch die Heizeinrichtung ist, und sich während des gesamten Denaturierungsschritts auf zumindest 10 % optional jedoch auf der gesamten Kontaktfläche der Heizeinrichtung bzw. des Lokalheizelements bzw. der Lokalheizelemente mit dem Reaktionsvolumen kein zeitlich stabiler Temperaturgradient einstellt, also ein Nicht-Gleichgewichts-Zustand vorliegt.

Ein Temperaturgradient gilt nach einer Dauer t1 nach dem Beginn der Erwärmung durch das Lokalheizelement als "zeitlich stabil" im Sinne der vorliegenden Offenbarung, wenn sich der Betrag seiner maximalen Steigung zu einem Zeitpunkt 2t1 um weniger als 30 % gegenüber dem Betrag seiner maximalen Steigung zum Zeitpunkt t1 geändert hat.

Dabei ist für die Feststellung der zeitlichen Stabilität lediglich der Vergleich der Beträge der maximalen Steigung relevant, nicht jedoch ob die Heizeinrichtung bzw. das Lokalheizelement bzw. die Lokalheizelemente zum Zeitpunkt 2t1 Wärme erzeugt oder nicht. Optional hat sich der Betrag der Steigung zum Zeitpunkt 2t1 um weniger als 20 %, weiter optional um weniger als 15 %, mehr optional um weniger als 10 %, besonders optional um weniger als 5 % gegenüber dem Betrag seiner maximalen Steigung zum Zeitpunkt t1 geändert. Der Gradient hat seine maximale Steigung in der Regel an der Oberfläche des Lokalheizelements bzw. der Lokalheizelemente.

Während sich mit manchen Geometrien auch im stationären Gleichgewichtszustand steile Temperaturgradienten realisieren lassen, ermöglicht das lokale Heizen durch die Nutzung der Nicht-Gleichgewichts-Zustände, d.h. der Zeit des Heizvorgangs, bevor sich überhaupt ein zeitlich stabiler TemperaturGradient ausgebildet hat, ein Heizen mit besonders wenig Energie. Während im stationären Gleichgewichtszustand besonders steile Temperaturgradienten dann entstehen können, wenn ein starker Wärme- und damit Energieabfluss erfolgt, kann bei der Nutzung der Nicht-Gleichgewichtszustände ein starker Temperaturgradient bereits mit sehr geringen Energiemengen auch dann erreicht werden, wenn der Wärmeabfluss aus dem Reaktionsvolumen gering ist. Somit ermöglicht das lokale Heizen steile Temperaturgradienten und entsprechend ein schnelles Aufheizen der Aufheizzone, ohne einen signifikanten Energieeintrag und ein damit verbundenes Aufheizen der gesamten Reaktionslösung zu benötigen.

Ein Vorteil durch die kurzen Heiz- und Denaturierungsschritte besteht darin, dass die im Denaturierungsschritt erforderliche Energiemenge so klein ist, dass auf eine aktive Kühlung (zur Rückkehr auf die Elongations- bzw. Annealing-Temperatur) verzichtet werden kannn, da der geringe Wärmeeintraag im Reaktionsvolumen und dessen Umgebung dissipiert werden kann. D.h. gemäß einer Ausführungsform erfolgt die Abkühlung von der Denaturierungs- auf die Elongations- bzw. Annealingtemperatur passiv, durch Wärmediffusion, ohne aktive Kühlung d.h. optional ohne spezielle Kühlvorrichtung.

Die Denaturierung und optional auch andere Schritte der Nukleinsäure-Vervielfältigung bzw. PCR können somit lokal in der direkten Umgebung der Lokalheizelemente stattfinden, wobei optional mindestens einer der benötigten Primer an die magnetischen Mikropartikel funktionalisiert ist und mittels diesem in die Aufheizzone nahe der Heizeinrichtung bzw. dem Lokalheizelement bzw. an einem der Lokalheizelemente angelagert werden, um auch das Amplikon dort entstehen zu lassen und somit eine Denaturierung bei lokaler Erwärmung zu ermöglichen. Mit anderen Worten, dadurch, dass aufgrund des Anlagerns der Mikropartikel an dem Lokalheizelement optional eine Lokalisierung von Schritten der PCR, insbesondere Hybridisierung, Elongation und/oder Denaturierung, sowie optional auch die Erzeugung eines Signals zum Beobachten des Fortschritts der PCR in unmittelbarer Nähe des Lokalheizelements erreicht wird, kann das Erwärmen des Reaktionsvolumens auf einen Bruchteil des Reaktionsvolumens beschränkt werden.

Die Kombination eines Verfahrens zur Extraktion einer Target-Nukleinsäure und/oder zum Vervielfältigen einer Target-Nukleinsäure gemäß einer Ausführungsform kann somit in besonders vorteilhafter Weise mit einer Vorrichtung zur Durchführung einer PCR auf Basis von lokalem Heizen kombiniert werden. Ein besonderer Vorteil kann sich daraus ergeben, dass die Target-Nukleinsäure bereits mittels der angelagerten magentischen Mikropartikeln an dem Lokalheizelement angeordnet ist und sich die Target-Nukleinsäure demnach bereits in der Aufheizzone befindet, welche mittels lokalem Heizen auf die Denaturierunstemperatur oder darüber hinaus erhitzt werden kann. Besonders optional sind die magnetischen Mikropartikel mit Primern für die PCR funktionalisiert, wodurch erreicht werden kann, dass die am Heizelement angeordneten Target-Nukleinsäuren bereits mit einem Primer hybridisiert sind.

Die PCR nutzt optional mindestens zwei Oligonukleotide, die als "Primer" bezeichnet werden, einen Vorwärtsprimer (auch als "Forward Primer" bezeichnet) und einen Rückwärtsprimer (auch als "Reverse Primer" bezeichnet). Der Vorwärtsprimer ist komplementär zum 3'-Ende des Originals und der Rückwärtsprimer ist komplementär zum 3'-Ende des Komplements. Im Hybridisierungsschritt (auch als "Annealingschritt" bezeichnet) hybridisiert der Vorwärtsprimer und/oder der Rückwärtsprimer an eine ihm komplementäre Sequenz im Original bzw. Komplement bzw. Amplikon. Der Hybridisierungsschritt findet gewöhnlich bei einer Temperatur statt, die ein Hybridisieren der Vorwärts- und Rückwärtsprimer an deren komplementäre Sequenzen im Original bzw. Komplement bzw. Amplikon hervorruft oder zumindest begünstigt. Sie wird optional so gewählt, dass sie ein möglichst spezifisches Hybridisieren der Primer ermöglicht. Die Hybridisierungstemperatur liegt typischerweise zwischen 50° C und 72° C. Einer oder mehrere der Primer können optional an die magnetischen Mikropartikel funktionalisiert sein. Optional können entweder die Vorwärts-Primer oder die Rückwärtsprimer an die magnetischen Mikropartikel funktionalisiert sein. Alternativ oder zusätzlich können zumindest manche mangetische Mikropartikel auch mit Vorwärts- und Rückwärtsprimern funktionalisiert sein.

Im Elongationsschritt werden die hybridisierten Primer von einem Polymerase-Enzym komplementär verlängert. So kann ausgehend von dem Vorwärtsprimer ein Komplement und ausgehend von dem Rückwärtsprimer ein Original synthetisiert werden. Zum Zweck der Elongation wird die Polymerase einer Temperatur ausgesetzt, die eine Elongation ermöglicht oder zumindest begünstigt. Bei der Verwendung einer Polymerase von dem Bakterium Thermus aquaticus (Taq) wird typischerweise eine Elongationstemperatur von 72 ° C verwendet. In manchen Ausführungsformen der PCR sind die Hybridisierungs- und die Elongationstemperaturen identisch, das heißt, beide Schritte finden bei der gleichen Temperatur statt (das heißt, es gibt nur zwei Temperaturstufen während der PCR, eine kombinierte Hybridisierungs- und Elongationstemperatur und eine Denaturierungstemperatur).

Die Denaturierungstemperatur entspricht einer Temperatur, bei welcher ein Nukleinsäure-Doppelstrang denaturiert wird, d.h. bei welcher der Nukleinsäure-Doppelstrang in seine beiden Einzelstränge aufgetrennt wird. So kann in dem Denaturierungsschritt zum Beispiel die Extraktionsnukleinsäure von der daran hybridisierten Nukleinsäure getrennt werden. Eine optionale Art der Denaturierung ist eine thermische Denaturierung (auch als "Schmelzen" bezeichnet). Dazu wird zumindest ein Teil des Nukleinsäure-Doppelstrangs oder der ganze Doppelstrang einer Temperatur, welche gleich oder höher als die Denaturierungstemperatur ist, ausgesetzt, die ein Trennen der Nukleinsäure-Doppelstränge hervorruft oder zumindest begünstigt. Die optionale Denaturierungstemperatur ist einerseits so hoch gewählt, dass Nukleinsäure-Doppelstränge aufgetrennt werden können. Andererseits ist die optionale Denaturierungstemperatur so niedrig gewählt, dass eine DNA-Polymerase, die sich möglicherweise ebenfalls in der Reaktionslösung befindet, nicht wesentlich geschädigt wird. Ein typischer Wert für die Denaturierungstemperatur kann beispielsweise bei 95 °C liegen. Das Beheizen des Lokalheizelements auf eine Temperatur, welche gleich oder größer der Denaturierungstemperatur ist, kann optional den Vorteil bieten, dass sich die Target-Nukleinsäure zumindest teilweise von der Extraktionsnukleinsäure löst und frei in die Reaktionslösung übergeht. Dies kann beispielsweise für eine anschließend durchzuführende Amplifikation der extrahierten Nukleinsäure vorteilhaft sein und/oder wenn die Target-Nukleinsäure von dem Lokalheizelement entfernt bzw. getrennt werden soll.

Optional umfasst das Verfahren zur Extraktion der Target-Nukleinsäure auf die Probenflüssigkeit ferner ein Entfernen der Probenflüssigkeit aus dem Reaktionsgefäß und ein Bereitstellen einer Extraktionslösung in dem Reaktionsgefäß derart, dass die an das Extraktionselement angelagerten magnetischen Mikropartikel mit der daran hybridisierten Target-Nukleinsäure zumindest teilweise in der Extraktionslösung suspendiert werden. Dadurch, dass die magnetischen Mikropartikel und die damit verbundenen Target-Nukleinsäuren an einem Extraktionselement, optional etwa an einer Gefäßwand des Reaktionsgefäßes, angelagert sind, verbleiben diese im Reaktionsgefäß, während der nicht mehr benötigte Rest der Probenflüssigkeit aus dem Reaktionsgefäß entfernt werden kann. Durch das Einfüllen der Extraktionslösung können die an der Gefäßwand sedimentierten magnetischen Mikropartikel wieder suspendiert werden und stehen somit für die weitere Verwendung in der Extraktionslösung bereit. Um ein zuverlässiges Suspendieren der magnetischen Mikropartikel in der Extraktionslösung zu erzielen, kann es vorteilhaft sein, durch unterstützende mechanische Krafteinwirkung das Lösen der angelagerten Mikropartikel zu unterstützen, beispielsweise durch ein Schütteln und/oder Vibrieren, etwa mittels eines Vortexmischers, und/oder durch ein Rühren, etwa mittels eines magnetischen Rührstabs, und/oder durch eine Beaufschlagung der Extraktionslösung und/oder des Reaktionsgefäßes mit Ultraschall. Die mechanische Krafteinwirkung kann zudem optional für ein Mischen der Probenflüssigkeit und/oder Reaktionslösung und oder für den Waschschritt verwendet werden. Alternativ oder zusätzlich zur mechanischen Krafteinwirkung kann zudem optional ein veränderliches Magnetfeld für eine Durchmischung der magnetischen Mikropartikel in der Reaktionslösung und/oder in der Probenflüssigkeit verwendet werden.

Optional wird zwischen dem Entfernen der Probenflüssigkeit und dem Bereitstellen der Extraktionslösung in dem Reaktionsgefäß das Reaktionsgefäß einem oder mehreren Waschschritten unterzogen. Es kann dahingehend vorteilhaft sein, dass nach dem Entfernen der Probenflüssigkeit etwaige verbleibende Reste der Probenflüssigkeit und/oder andere Verschmutzungen im Reaktionsgefäß entfernt werden können und auf diese Weise mögliche Beeinträchtigungen der beabsichtigten Verwendung der extrahierten Target-Nukleinsäure reduziert oder vermieden werden können.

Optional ist das Extraktionselement zumindest teilweise aus ferromagnetischem Material ausgebildet. Dies ermöglicht optional die Bereitstellung des Magnetfeldes mittels einer Wechselwirkung des ferromagnetischen Extraktionselements mit den magnetischen Mikropartikeln. Optional ist bei einer derartigen Ausgestaltung kein weiterer Magnet erforderlich, um das für die Anlagerung der Mikropartikel an das Extraktionselement erforderliche Magnetfeld bereitzustellen. Optional weist das Extraktionselement eine Folie und/oder einen Draht auf, oder ist als Folie oder Draht ausgebildet. Optional ist das Extraktionselement an einer Gefäßwand des Reaktionsgefäßes ausgebildet und/oder bildet einen Teil einer Gefäßwand des Reaktionsgefäßes bildet.

Optional umfasst das Verfahren zur Amplifikation der Target-Nukleinsäure ferner ein Aussetzen zumindest eines Teils der am Lokalheizelement angelagerten magnetischen Mikropartikeln einem Magnetfeld derart, dass die magnetischen Mikropartikel den lokal erhitzten Bereich, d.h. die Aufheizzone, der Reaktionslösung verlassen und in der Reaktionslösung suspendiert werden. Mit anderen Worten wird ein Magnetfeld bzw. eine magnetische Kraft zusätzlich dazu genutzt, um die an dem Lokalheizelement abgelagerten magnetischen Mikropartikel wieder in der Reaktionslösung zu suspendieren, sodass diese in der Reaktionslösung optional mit weiteren Target-Nukleinsäuren oder Primern binden können und elongiert werden können, um ein weiteres Amplikon zu erzeugen. Dies wird insbesondere dadurch ermöglicht, wenn während des lokalen Erhitzens im Bereich des Lokalheizelements, während die magnetischen Mikropartikel an diesen angelagert sind, die an die Primer der magnetischen Mikropartikel hybridisierten Target-Nukleinsäuren denaturiert werden und sich von den Primern lösen und daraufhin die Primer wieder einzelsträngig vorliegen und prinzipiell für eine Hybridisierung mit einer weiteren Target-Nukleinsäure zur Verfügung stehen.

Optional entspricht die Temperatur der Reaktionslösung außerhalb des lokal erhitzten Bereichs, d.h. der Aufheizzone, im Wesentlichen der Hybridisierungstemperatur der Target-Nukleinsäure. Dies bietet den Vorteil, dass außerhalb des lokal erhitzten bzw. beheizten Bereichs eine Hybridisierung von einzelsträngigen Primern mit einzelsträngigen Target-Nukleinsäuren und die Elongation ermöglicht werden. Dies kann insbesondere dann vorteilhaft sein, wenn, wie oben beschrieben, ein Magnetfeld bzw. eine magnetische Kraft genutzt wird, um die an das Lokalheizelement angelagerten magnetischen Mikropartikeln von dem Lokalheizelement abzustoßen, sodass diese wieder in der Reaktionslösung suspendiert werden. Die Hybridisierungstemperatur kann optional einen Hybridisierungstemperaturbereich darstellen. Beispielsweise können verschiedene einzelne Hybridisierungstemperaturen verwendet werden, um im Rahmen eines Multiplexings in einer Reaktionslösung mehrere verschiedene Target-Nukleinsäuren zu amplifizieren, wobei verschiedenen Target-Nukleinsäuren optional unterschiedliche Hybridisierungstemperaturen aufweisen, wobei die verschiedenen Hybridisierungstemperaturen in dem Hybridisierungstemperaturbereich liegen.

Optional bleibt beim lokalen Erhitzen des Bereichs der Reaktionslösung, in welchem die magnetischen Mikropartikel angelagert sind, die Reaktionslösung außerhalb des lokal erhitzten Bereichs im Wesentlichen isothermal. "Im Wesentlichen isothermal" bedeutet dabei, dass die Reaktionslösung außerhalb des lokal erhitzten Bereichs kein Temperatur Cycling erfährt und insbesondere nicht die Denaturierungstemperatur erreicht. Optional bleibt die Reaktionslösung außerhalb des lokal erhitzten Bereichs in einem Temperaturbereich zwischen der optionalen Annealingtemperatur und einer Temperatur, welche etwa 10 °C über der optionalen Annealingtemperatur liegt. Dies bietet den Vorteil, dass keine Kühlung der Reaktionslösung, insbesondere keine aktive Kühlvorrichtung, für das Kühlen der Reaktionslösung bereitgestellt werden muss. Außerdem bietet dies den Vorteil, dass die Enzyme in der Reaktionslösung in weiten Teilen der Reaktionslösung keinen hohen Temperaturen und keinen hohen Temperaturunterschieden ausgesetzt werden müssen.

Optional ist eine Vorrichtung zur Amplifikation der Target-Nukleinsäure derart ausgestaltet, dass die Temperatur der Reaktionslösung außerhalb des lokal beheizten Bereichs lediglich passiv reduziert wird. Mit anderen Worten ist die Vorrichtung derart ausgestaltet, dass kein aktives Kühlen der Reaktionslösung, beispielsweise mittels eines Peltier-Elements und/oder eines Kühlkompressors, erfolgt. Vielmehr wird der Reaktionslösung lediglich durch eine natürliche Wärmeabgabe an die Umgebung gekühlt, ohne dass der Reaktionslösung durch die künstliche Herbeiführung eines erhöhten Temperaturgradienten zwischen der Reaktionslösung und ihrer Umgebung herbeigeführt wird. Dies bietet den Vorteil, dass keine aktive Kühleinrichtung und insbesondere keine Stromversorgung für eine aktive Kühlvorrichtung bereitgestellt werden muss.

Entsprechend wird auch in einem Verfahren zu Amplifikation einer Target-Nukleinsäure die Reaktionslösung lediglich durch eine Wärmeabgabe an die Umgebung der Reaktionslösung gekühlt. Dabei erfolgt keine aktive Kühlung der Reaktionslösung und der direkten Umgebung der Reaktionslösung, mit der die Reaktionslösung in direktem thermischem Kontakt steht.

Optional können mittels des Verfahrens auch mehrere verschiedene Target-Nukleinsäuren in einer Reaktionslösung amplifiziert werden. Dadurch kann ein Multiplexing erreicht werden. Optional können dabei verschiedenartige Primer für verschiedene Target-Nukleinsäuren an einem Mikropartikel angebracht sein. Alternativ oder zusätzlich können Mikropartikel bereitgestellt werden, welche jeweils nur eine Art von Primern für eine Target-Nukleinsäure aufweisen.

Die Denaturierungstemperatur kann optional einen Denaturierungstemperaturbereich darstellen. Beispielsweise können verschiedene einzelne Denaturierungstemperaturen verwendet werden, um im Rahmen eines Multiplexings in einer Reaktionslösung mehrere verschiedene Target-Nukleinsäuren zu amplifizieren, wobei verschiedenen Target-Nukleinsäuren optional unterschiedliche Denaturierungstemperaturen aufweisen, wobei die verschiedenen Denaturierungstemperaturen in dem Denaturierungstemperaturbereich liegen.

Optional erfolgt das Bereitstellen der Reaktionslösung im Reaktionsgefäß in Schritt f) derart, dass die an das Lokalheizelement angelagerten magnetischen Mikropartikel mit der daran hybridisierten Target-Nukleinsäure zumindest teilweise in der Reaktionslösung suspendiert werden. Dies bietet den Vorteil, dass die Mikropartikeln wieder in der Reaktionslösung verteilt werden können und dort optional mit weiteren Target-Nukleinsäuren hybridisieren können.

Optional umfasst Schritt c) des oben bezeichneten Verfahrens zur Amplifikation einer Target-Nukleinsäure ein Temperieren zumindest eines Teils der Probenflüssigkeit auf die Hybridisierungstemperatur der Target-Nukleinsäure. Dies ermöglicht ein effizientes Hybridisieren der in der Probenflüssigkeit befindlichen einzelsträngig vorliegenden Target-Nukleinsäuren oder (Reverse-) Primern mit den einzelsträngig vorliegenden Extraktionsnukleinsäuren bzw. Primern, die auf die magnetischen Mikropartikel funktionalisiert sind.

Optional wird zwischen den Schritten e) und f) des oben bezeichneten Amplifikationsverfahrens das Reaktionsgefäß einem oder mehreren Waschschritten unterzogen. Dies bietet den Vorteil, dass etwaige Verunreinigungen und/oder Reste der Probenflüssigkeit entfernt werden können, welche für die Durchführung der anschließenden Amplifikationsreaktion unerwünscht sind.

Optional wird der Bereich, der in Schritt g) des oben bezeichneten Amplifikationsverfahrens mittels des Lokalheizelements erhitzt wird, d.h. die Aufheizzone, in mehreren aufeinanderfolgenden Heizschritten jeweils auf die Denaturierungstemperatur erhitzt und zwischen den Heizschritten im Wesentlichen auf die Hybridisierungstemperatur abkühlt. "Im Wesentlichen auf die Hybridisierungstemperatur abgekühlt" bedeutet dabei, dass die optimale Hybridisierungstemperatur nicht exakt erreicht werden muss, sondern dass vielmehr ein Temperaturbereich ausreichend sein kann, in welchem eine Hybridisierung und/oder Elongation mit ausreichend hoher Effizienz erfolgen kann. Das Abkühlen auf die Hybridisierungstemperatur kann insbesondere selbstständig erfolgen, durch eine Diffusion der beim Heizen eingebrachten Wärme von dem beheizten Bereich in die restliche Reaktionslösung, welche auf diese Weise als ein Wärmereservoir bzw. Kältereservoir dient. Dabei kann es vorteilhaft sein, wenn die restliche Reaktionslösung im Wesentlichen auf der Hybridisierungstemperatur gehalten wird, beispielsweise mittels eines externen Heizblocks.

Optional erfolgt mittels der mehreren Heizschritte und der dazwischen liegenden Abkühlungsschritte im mittels des Lokalheizelement beheizten Bereich eine Amplifikation der Target-Nukleinsäure mittels einer PCR. Dies bietet den Vorteil, dass das Durchlaufen der verschiedenen Temperaturstufen bzw. das thermische Cyclen in besonders kurzer Zeit durchgeführt werden kann, da für das Erhitzen auf die Denaturierungstemperatur und das Abkühlen auf die Hybridisierungstemperatur aufgrund der Verwendung des lokalen Heizens nur eine sehr geringe Zeitdauer erforderlich ist.

Optional umfasst das Lokalheizelement eine oder mehrere elektrisch beheizbare Metallfolien oder ist als solche ausgebildet. Dies bietet den Vorteil, dass das Lokalheizelement ein großes Flächen-Volumen-Verhältnis aufweisen kann und somit eine große Heizfläche bei gleichzeitig geringer Wärmekapazität bereitstellen kann. Dadurch kann zum einen ein großer beheizbarer Bereich bereitgestellt werden und zum anderen die für das Erhitzen und Abkühlen des beheizten Bereichs und des Lokalheizelements erforderliche Zeitdauer bzw. dessen thermische Trägheit minimiert werden.

Optional weist der mittels des Lokalheizelements erhitzte bzw. beheizte Bereich der Reaktionslösung einen sich vom Lokalheizelement während des Erhitzens weg erstreckenden Temperaturgradienten auf, wobei sich der Betrag des Temperaturgradienten optional auf einer Länge zwischen 1 $\mu$m und 10$\mu$m von der Oberfläche des Lokalheizelements halbiert. Die Dicke des beheizten Bereichs bzw. die Länge, über welche sich der beheizte Bereich von dem Lokalheizelement in die Reaktionslösung erstreckt, ist dabei optional derart zu wählen, dass sich die an dem Lokalheizelement angelagerten magnetischen Mikropartikel und die daran gebundenen Target-Nukleinsäuren in diesem beheizten Bereich befinden, aber dennoch das Volumen des beheizten Bereichs möglichst klein im Verhältnis zum Volumen der restlichen Reaktionslösung ist, um ein möglichst großes Wärme- bzw. Kältereservoir bereitzustellen.

Optional ist der Magnet zur Erzeugung eines veränderbaren Magnetfeldes ausgebildet, sodass das veränderbare Magnetfeld in einem ersten Zustand auf zumindest einen Teil der in der Reaktionslösung befindlichen magnetischen Mikropartikel derart wirkt, dass sich diese an dem Lokalheizelement anlagern und in einem zweiten Zustand auf die an das Lokalheizelement angelagerten magnetischen Mikropartikel derart wirkt, dass diese das Lokalheizelement verlassen und in der Reaktionslösung suspendiert werden. Dadurch können die magnetischen Mikropartikel optional mehrmals am Lokalheizelement angelagert und wieder davon abgestoßen werden, sodass diese in der Reaktionslösung mit weiteren Target-Nukleinsäuren hybridisieren können.

Optional bildet das Lokalheizelement zumindest einen Teil einer Gefäßwand des Reaktionsgefäßes. Dies bietet den Vorteil, dass auf einfache Weise ein direkter Kontakt zwischen dem Lokalheizelement und der Probenflüssigkeit bzw. der Reaktionslösung hergestellt werden kann. Ferner bietet dies die Möglichkeit, die Vorrichtung bzw. das Reaktionsgefäß mit geringem Herstellungsaufwand herzustellen und dadurch die Herstellungskosten gering zu halten.

Optional weist der Magnet oder die mehreren Magnete einen in seiner Position und/oder Orientierung relativ zum Reaktionsgefäß veränderbaren Permanentmagneten und/oder einen Elektromagneten auf. Beispielsweise kann bei Verwendung eines Permanentmagneten das Magnetfeld dadurch geändert werden, dass eine Orientierung des Permanentmagneten zum Reaktionsgefäß und/oder ein Abstand des Permanentmagneten vom Reaktionsgefäß verändert wird. Auch kann die Richtung des Magnetfelds beispielsweise dadurch geändert werden, dass der Permanentmagnet relativ zum Reaktionsgefäß umgedreht wird, sodass beispielsweise die zum Reaktionsgefäß gewandte Seite des Permanentmagneten vom magnetischen Nordpol zum Südpol des Permanentmagneten wechselt bzw. umgekehrt. Bei der Verwendung eines Elektromagneten kann das veränderbare Magnetfeld beispielsweise durch eine Änderung des Stromflusses, wie etwa der Stromstärke und/oder der Stromflussrichtung, geändert werden. Beispielsweise kann der Elektromagnet eine oder mehrere Magnetspulen und optional einen ferromagnetischen Kern umfassen. Optional ist der Magnet auf einer dem Reaktionsgefäß abgewandten Seite des Lokalheizelements ausgebildet. Dies bietet den Vorteil, dass durch den Permanentmagneten in dieser Anordnung ein Anziehen der magnetischen Mikropartikel auf das Lokalheizelement besonders effektiv und einfach erzielt werden kann. Alternativ oder zusätzlich können der eine oder die mehreren Magnete in ihrer Position relativ zum Reaktionsgefäß geändert werden, um ein veränderbares Magnetfeld in der Reaktionslösung zu erzielen. Alternativ oder zusätzlich können auch mehrere unterschiedlich gepolte Magneten an das Reaktionsgefäß herangeführt werden, um in der Reaktionslösung ein veränderbares Magnetfeld zu erzielen.

Geeignete Parameter für die Durchführung einer Amplifikationsreaktion und insbesondere einer PCR, insbesondere hinsichtlich geeigneter Inhaltsstoffe der Reaktionslösung, können beispielsweise der Druckschrift DE102016120124A1 entnommen werden. Auch können der DE102016120124A1 beispielhafte Angaben hinsichtlich der Temperaturen und Zeitdauern für das lokale Heizen entnommen werden, sodass diesbezüglich auf die bereits veröffentlichte Druckschrift verwiesen wird und die Offenbarung der genannten Druckschrift als von der Offenbarung der vorliegenden Offenbarung umfasst anzusehen ist.

Die oben genannten und im Folgenden erläuterten Merkmale und Ausführungsformen und Beispiele sind dabei nicht nur als in den jeweils explizit genannten Kombinationen offenbart anzusehen, sondern sind auch in anderen technisch sinnhaften Kombinationen und Ausführungsformen vom Offenbarungsgehalt umfasst.

Weitere Einzelheiten und Vorteile sollen nun anhand der folgenden Beispiele und optionalen Ausführungsformen mit Bezug auf die Figuren näher erläutert werden.

Es zeigen:

Figur 1      ein funktionalisiertes magnetisches Mikropartikel gemäß einer optionalen Ausführungsform;

Figur 2      ein Reaktionsgefäß gemäß einer optionalen Ausführungsform in einer schematischen Darstellung;

Figuren 3A bis 3D      Erläuterungen eines Verfahrens zur Extraktion einer Target-Nukleinsäure gemäß einer optionalen Ausführungsform;

Figuren 4A bis 4D      Erläuterungen eines Verfahrens zur Amplifikation einer Target-Nukleinsäure gemäß einer optionalen Ausführungsform;

Figuren 5A bis 5H      Erläuterungen eines Verfahrens zur Extraktion und Amplifikation einer Target-Nukleinsäure gemäß einer optionalen Ausführungsform;

Figuren 6A bis 6C      Erläuterungen zu einem Funktionsprinzip einer Amplifikation einer Target-Nukleinsäure gemäß einer optionalen Ausführungsform.

In den folgenden Figuren werden gleiche oder ähnliche Elemente in den verschiedenen Ausführungsformen der Einfachheit halber mit gleichen Bezugszeichen bezeichnet.

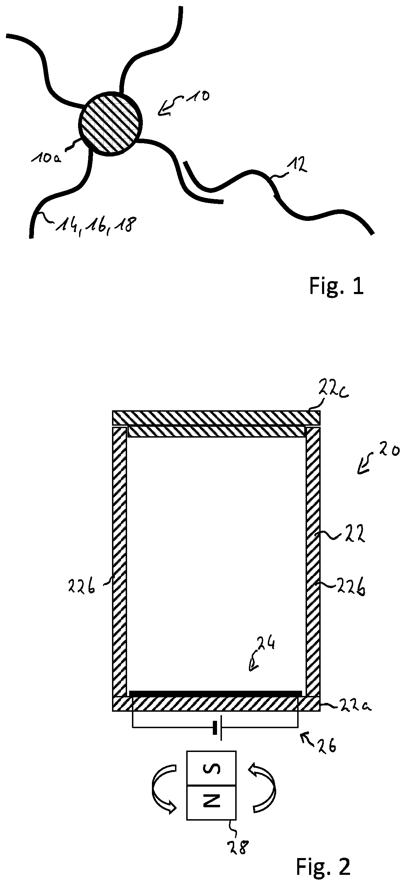

Figur 1 zeigt in einer schematischen Darstellung ein magnetisches Mikropartikel 10, welches in einem Verfahren zur Extraktion und/oder Amplifikation einer Target-Nukleinsäure 12 gemäß einer optionalen Ausführungsform verwendet werden kann. Das magnetische Mikropartikel 10 ist dabei zumindest teilweise aus einem ferromagnetischen Material ausgebildet, wodurch die magnetischen Eigenschaften des Mikropartikels bereitgestellt werden. Das Mikropartikel 10 ist gemäß der gezeigten optionalen Ausführungsform kugelförmig ausgebildet, wenngleich gemäß anderen Ausführungsformen auch anderweitige Formen möglich sind. Die Größe des Mikropartikels bzw. dessen Durchmesser liegt in einem Bereich von 1 μm.

Außerdem weist das Mikropartikel 10 gemäß der gezeigten optionalen Ausführungsform eine Beschichtung 10a auf, welche die Funktionalisierung des Mikropartikels 10 mit Nukleinsäuren ermöglicht oder erleichtert. Die Beschichtung kann dabei aus Streptavidin oder Biotin bestehen oder diese umfassen und ermöglicht oder erleichtert dadurch das Funktionalisieren des Mikropartikels mit Nukleinsäuren mittels einer Streptavidin-Biotin-Verbindung. Optional ist dabei Streptavidin am Mikropartikel und Biotin an den für die Funktionalisierung vorgesehenen Primern angebracht, wenngleich auch eine entgegengesetzte Anordnung möglich ist.

In der gezeigten schematischen Darstellung sind vier Oligonukleotide 14 zu erkennen, welche an das magnetische Mikropartikel 10 funktionalisiert sind. Zur besseren Übersichtlichkeit sind lediglich vier Oligonukleotide dargestellt, wenngleich die tatsächliche Besetzungsdichte der Oberfläche des Mikropartikels mit Oligonukleotiden deutlich größer sein kann. Auch können die tatsächlichen Größenverhältnisse der Oligonukleotide 14 und der Nukleinsäuren im Allgemeinen zum Mikropartikel 10 deutlich von der gezeigten Darstellung abweichen. Zur Verwendung des Mikropartikels 10 in einem Verfahren zur Extraktion einer Target-Nukleinsäure 12 können die Oligonukleotide als Extraktionsnukleinsäuren 16 ausgebildet sein. Zur Verwendung in einem Verfahren zur Amplifikation einer Target-Nukleinsäure 12 können die Oligonukleotide 14 als Primer 18 ausgebildet sein. Gemäß optionalen Ausführungsformen von Verfahren, welche der Extraktion und Amplifikation einer Target-Nukleinsäure 12 dienen, können die Oligonukleotide 14 als Primer 18 ausgebildet sein und zugleich die Funktion einer Extraktionsnukleinsäure 16 innehaben. Die Oligonukleotide 14 können dabei optional zwei oder mehr Abschnitte aufweisen. Beispielsweise kann ein erster Abschnitt als Funktionalisierungsabschnitt ausgebildet sein, mittels welchem das Oligonukleotid an das Mikropartikel 10 funktionalisiert ist. Ein zweiter Abschnitt kann die Funktionalität des Oligonukleotids 10 als Primer 18 und/oder Extraktionsnukleinsäure 16 bereitstellen.

In der gezeigten Darstellung ist an einen der Oligonukleotide 14 eine Target-Nukleinsäure 12 hybridisiert bzw. gebunden. Auf diese Weise ist die Target-Nukleinsäure 12 über das Oligonukleotid 14 fest mit dem magnetischen Mikropartikel 10 verbunden und verbleibt insbesondere auch dann mit dem Mikropartikel 10 verbunden, wenn das Mikropartikel 10 aus einer Probenflüssigkeit entfernt wird. Hingegen kann die Target-Nukleinsäure 12 dadurch wieder vom Oligonukleotid 14 und somit vom Mikropartikel 10 entfernt werden, indem mittels einer Denaturierung die Hybridisierung der Target-Nukleinsäure 12 mit dem Oligonukleotid aufgelöst wird.

Figur 2 zeigt in einer schematischen Darstellung eine Vorrichtung 20 gemäß einer optionalen Ausführungsform zur Amplifikation einer Target-Nukleinsäure 12. Die Vorrichtung 20 umfasst dabei ein Reaktionsgefäß 22, ein Lokalheizelement 24 und einen Magneten 26. Das Reaktionsgefäß 22 weist wiederum einen Boden 22a, eine umlaufende Wand 22b oder mehrere Wände 22b und einen Deckel 22c auf. Der Deckel 22c ist gemäß der gezeigten Ausführungsform vom restlichen Reaktionsgefäß 22 entfernbar, um etwa eine Probenflüssigkeit oder eine Reaktionslösung in das Reaktionsgefäß 22 einzubringen oder aus diesem zu entfernen. Das Reaktionsgefäß 22 kann unabhängig von dem Lokalheizelement 24 temperierbar sein, um etwa einen darin befindlichen Inhalt auf eine Hybridisierungstemperatur zu bringen oder bei dieser zu halten. Der Deckel 22c kann dabei separat vom restlichen Reaktionsgefäß 22 temperierbar sein, etwa um eine Kondensation von Flüssigkeit an der Innenseite des Deckels 22c zu vermeiden. Optional ist das Reaktionsgefäß zumindest teilweise transparent ausgebildet, um eine Sichtprüfung des Inhalts von außen vornehmen zu können, ohne das Reaktionsgefäß 22 zu öffnen und/oder um eine optische Messung des Inhalts durch das Reaktionsgefäß 22 hindurch durchführen zu können. Eine derartige optische Messung kann beispielsweise zur Durchführung einer Amplifikationsreaktion vorteilhaft sein, insbesondere wenn zur Detektion der Amplikons Kombinationen aus Farbstoffen und Quenchern verwendet werden und die erfolgreiche Amplifikation anhand eines optischen Signals dieser Farbstoffe durchgeführt wird.

Das Lokalheizelement 24 ist optional als eine Metallfolie ausgebildet, welche resistiv durch das Zuführen von elektrischem Strom beheizt bzw. erhitzt werden kann. Dabei ist die Metallfolie optional möglichst dünn ausgebildet, um trotz großer Oberfläche und dementsprechend großer Heizfläche ein geringes Volumen und damit einhergehend eine geringe Wärmekapazität aufzuweisen. Für die Leistungsversorgung zum Beheizen der Metallfolie weist das Lokalheizelement gemäß der gezeigten Ausführungsform eine Spannungsquelle 26 auf, mittels welcher der Metallfolie elektrische Energie zugeführt werden kann. Optional weist das Lokalheizelement bzw. die Metallfolie ein oder mehrere Löcher auf (nicht gezeigt), welche eine optische Messung des Inhalts in Transmission durch das Reaktionsgefäß 22 vom Deckel 22c aus durch den Innenraum bzw. Inhalt des Reaktionsgefäßes 22, durch das Loch oder die Löcher in dem Lokalheizelement 24 und durch den Boden 22a des Reaktionsgefäßes ermöglichen.

Außerdem weist die Vorrichtung 20 einen Magneten 28 auf, welcher gemäß der gezeigten Ausführungsform unterhalb des Reaktionsgefäßes 22 und insbesondere unterhalb des Lokalheizelements 24 angeordnet ist. Mittels des Magneten 28 kann im Innenraum des Reaktionsgefäßes 22 ein Magnetfeld bereitgestellt werden, um in dem Reaktionsgefäß 22 befindliche magnetische Mikropartikel 10 anzuziehen, sodass sich diese an das Lokalheizelement 24 anlagern, oder abzustoßen, sodass diese sich vom Lokalheizelement 24 lösen und von diesem entfernen. Der Magnet 28 kann optional als ein Permanentmagnet ausgebildet sein oder einen solchen umfassen und zur Umpolung oder Änderung des Magnetfeldes in seiner Orientierung relativ zum Reaktionsgefäß 22 geändert werden. Alternativ oder zusätzlich kann der Magnet 28 einen Elektromagneten umfassen, welcher durch eine Änderung des Stromflusses und der dadurch versursachten magnetischen Induktion ein Magnetfeld der gewünschten Stärke und/oder Polarität bereitstellt. Dabei ist es vorteilhaft, wenn das Reaktionsgefäß 22 zumindest teilweise keine ferromagnetischen und optional auch keine diamagnetischen Eigenschaften aufweist, um das Eindringen eines außerhalb des Reaktionsgefäßes 22 erzeugten Magnetfelds zu ermöglichen, ohne dieses in erheblichem Maße zu beeinflussen.

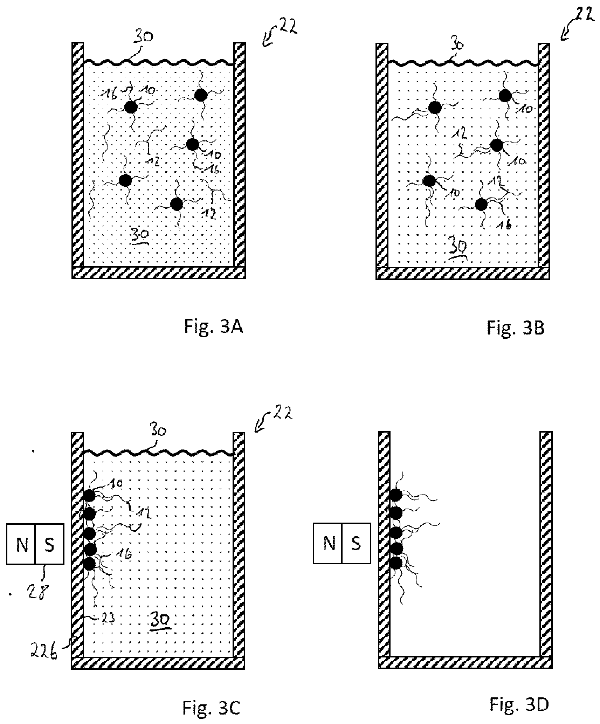

Anhand der Figuren 3A bis 3D wird im Folgenden ein Verfahren gemäß einer optionalen Ausführungsform zur Extraktion einer Target-Nukleinsäure 12 aus einer Probenflüssigkeit 30 erläutert. Zur Einfachheit halber ist in den schematischen Darstellungen das Reaktionsgefäß 22 ohne Deckel und ohne sonstiges optionales Zubehör gezeigt.

In dem Reaktionsgefäß 22 ist die Probenflüssigkeit 30 eingefüllt, in welcher sich mit Extraktionsnukleinsäure 16 funktionalisierte magnetische Mikropartikel 10 und die einzelsträngig vorliegende Target-Nukleinsäuren 12 frei bewegen. Außerdem können in der Probenflüssigkeit 30 noch weitere Elemente und Stoffe (nicht gezeigt) enthalten sein, die es jedoch bei der Extraktion von der Target-Nukleinsäure 12 zu trennen gilt. Beispielsweise können die in der Probenflüssigkeit 30 vorhandene einzelsträngige Target-Nukleinsäuren 12 durch herkömmliche Verfahren in die Probenflüssigkeit eingebracht worden sein, etwa durch Herauslösen von Bakterien und/oder Viren aus einem Rachenabstrich. Ebenso kann die Probenflüssigkeit 30 vor der Extraktion oder die Target-Nukleinsäuren 12 in anderem Umfeld vor der Extraktion einem Lyseprozess und/oder Denaturierungsprozess unterzogen worden sein, um die zu extrahierende Target-Nukleinsäuren 12 frei und einzelsträngig vorliegen zu haben.

In einem ersten Schritt, welcher in Figur 3A gezeigt ist, werden demnach die magnetischen Mikropartikel 10 in der Probenflüssigkeit 30 bereitgestellt, in welcher sich auch die Target-Nukleinsäuren 12 frei und einzelsträngig befinden.

Figur 3B zeigt einen zweiten Schritt, in dem zumindest ein Teil der Target-Nukleinsäuren 12 an die Extraktionsnukleinsäuren 16 der Mikropartikel 10 hybridisieren. Dazu kann es vorteilhaft sein, wenn die Probenflüssigkeit auf die Hybridisierungstemperatur, beispielsweise auf ca. 60° C, temperiert ist, um den Hybridisierungsvorgang zu begünstigen.

Im dritten Schritt, welcher in Figur 3C gezeigt ist, wird ein Magnetfeld im Inneren des Reaktionsgefäßes 22 bzw. in der Probenflüssigkeit 30 bereitgestellt. Gemäß der gezeigten Ausführungsform wird dies durch das Anordnen eines Magneten 28 außerhalb des Reaktionsgefäßes 22 erzielt, wobei der Magnet 28 außerhalb der linken Wand 22b des Reaktionsgefäßes angeordnet wird, sodass die in der Probenflüssigkeit 30 befindlichen magnetischen Mikropartikel 10 zumindest teilweise von diesem angezogen werden und sich an die Wand 22b des Reaktionsgefäßes 22 anlagern. Die Wand 22b des Reaktionsgefäßes 22 dient dabei als Extraktionselement 23. Gemäß anderen Ausführungsformen kann auch ein separates Extraktionselement bereitgestellt werden, welches beispielsweise in die Reaktionslösung 32 und in das Reaktionsgefäß 22 eingebracht werden kann.

Im vierten Schritt, der in Figur 3D gezeigt ist, wurde die Probenflüssigkeit 30 aus dem Reaktionsgefäß 22 entfernt, wobei die an der Wand des Reaktionsgefäßes angelagerten magnetischen Mikropartikel 10 und die daran gebundenen Target-Nukleinsäuren 12 nicht mit aus dem Reaktionsgefäß 22 entfernt wurden. Das Entfernen der Probenflüssigkeit aus dem Reaktionsgefäß 22 kann beispielsweise durch ein Abgießen und/oder Absaugen aus dem Reaktionsgefäß 22 erfolgen, wobei dabei eine möglichst geringen Sogwirkung vorteilhaft ist, um ein Lösen und Entfernen der an dem Reaktionsgefäß 22 angelagerten Mikropartikel 10 weitestgehend zu vermeiden. In Abhängigkeit von der Stärke des Anhaftens der sedimentierten magnetischen Mikropartikel 10 an dem Reaktionsgefäß 22 und von der beim Entfernen der Probenflüssigkeit 30 auftretenden Sogwirkung ist es nicht zwingend erforderlich, das Magnetfeld aufrecht zu erhalten, da die angelagerten Mikropartikel optional auch ohne Magnetfeld in dem angelagerten Zustand verbleiben.

Auch ist es nicht zwingend erforderlich, dass die Mikropartikel an einer Seitenwand 22b des Reaktionsgefäßes 2 anlagern. Auch kann bei entsprechendem Magnetfeld ein Anlagern an einer anderen Begrenzung des Reaktionsgefäßes 22 erfolgen, wie etwa am Boden 22a oder an der Innenseite des Deckels, sofern dieser in Kontakt mit der Probenflüssigkeit 30 steht.

Somit wurden mit diesem optionalen Verfahren die Target-Nukleinsäuren 12 mittels der magnetischen Mikropartikel aus der Probenflüssigkeit 30 extrahiert. Die Target-Nukleinsäuren 12 stehen dabei für eine weitere Verwendung zur Verfügung. Beispielsweise können diese durch Einfüllen einer anderen Flüssigkeit in das Reaktionsgefäß 22 wieder von der Gefäßwand 22b gelöst werden und wieder in der Flüssigkeit suspendiert werden.

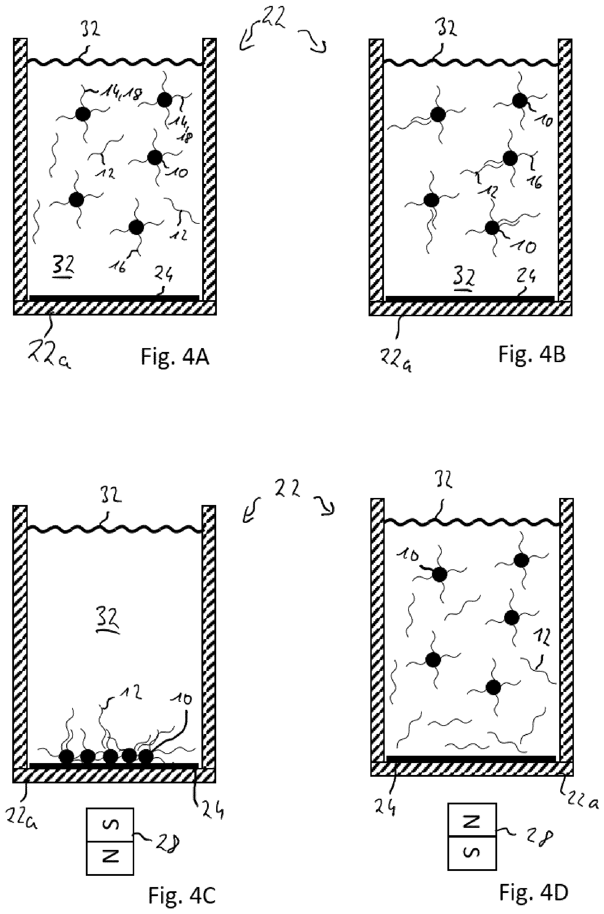

Anhand der Figuren 4A bis 4D wird im Folgenden ein Verfahren gemäß einer optionalen Ausführungsform zur Amplifikation von Target-Nukleinsäuren 12 erläutert. Die Amplifikation der Target-Nukleinsäuren 12 findet dabei in einem Reaktionsgefäß 22 statt, welches am Boden 22a ein Lokalheizelement 24 aufweist. Das Lokalheizelement 24 ist dabei als eine Metallfolie ausgebildet, welche sich an der Innenseite des Reaktionsgefäßes 22 über den Boden 22a des Reaktionsgefäßes 22 erstreckt.

Das Reaktionsgefäß 22 ist mit einer Reaktionslösung 32 befüllt (Figur 4A). Die Reaktionslösung 32 ist dazu ausgelegt, eine Amplifikationsreaktion für die Target-Nukleinsäure 12 durchzuführen, insbesondere mittels einer PCR. Dazu kann die Reaktionslösung 32 insbesondere die für die PCR notwendigen Inhaltsstoffe beinhalten, wie etwa die Nukleotide und/oder Enzyme und/oder Inhaltsstoffe einer Pufferlösung sowie weitere Primer, welche von den an die magnetischen Mikropartikel 10 funktionalisierten Primern 18 verschieden sind. Sind beispielsweise die an die magnetischen Mikropartikel 10 funktionalisierten Primer 18 als Vorwärts-Primer ausgebildet, können sich in der Reaktionslösung 32 frei bewegliche Rückwärtsprimer befinden. Sofern die Primer 18 an den magnetischen Mikropartikeln 18 als Rückwärtsprimer ausgebildet sind, können die zusätzlichen in der Reaktionslösung 32 vorliegenden Primer als Vorwärtsprimer ausgebildet sein. Sowohl die Target-Nukleinsäure 12 als auch die magnetischen Mikropartikel 10 sind in der Reaktionslösung 32 suspendiert und frei in der Reaktionslösung 32 beweglich. Die Oligonukleotide 14 sind dabei als Primer 18 ausgebildet, wobei die Primer 18 zumindest teilweise zu einem Sequenzabschnitt der Target-Nukleinsäure 12 komplementär sind und in der Amplifikationsreaktion als Primer dienen.

Die Reaktionslösung 32 wird dabei bei solchen Bedingungen bereitgestellt, welche eine Hybridisierung der Target-Nukleinsäuren 12 an die an die magnetischen Mikropartikel 10 funktionalisierten Primer 18 (Figur 4B) und eine Elongation der Primer durch eine in der Reaktionslösung 32 bereitgestellte Polymerase ermöglichen. Insbesondere können diese Bedingungen eine Temperierung der Reaktionslösung 32 auf eine Hybridisierungstemperatur umfassen. Außerdem können die Bedingungen weitere chemische und/oder biologische Bedingungen umfassen, welche insbesondere das Vorliegen der Target-Nukleinsäuren 12 in einzelsträngiger Form erlauben. Jene Target-Nukleinsäuren 12, welche mit einem Primer 18 hybridisiert sind, werden sodann zumindest teilweise durch eine Polymerase zu einem Doppelstrang elongiert, wodurch Amplikons Target-Nukleinsäure 12 generiert werden, welche sodann zunächst als Doppelstrang mit der Target-Nukleinsäure 12 an das magnetische Mikropartikel 10 gebunden sind.

Nach erfolgter Hybridisierung und Amplifikation wird sodann mittels eines Magneten 28 ein Magnetfeld im Reaktionsgefäß 22 bzw. in der Reaktionslösung 32 bereitgestellt, mittels welchem die magnetischen Mikropartikel 10 und die daran gebundenen Target-Nukleinsäuren 12 zum Lokalheizelement 24 bewegt werden, sodass diese sich zumindest teilweise dort anlagern (Figur 4C). Auf diese Weise erfolgt eine Konzentration der magnetischen Mikropartikel 10 und damit einhergehend eine Konzentration der gebundenen Target-Nukleinsäuren 12 an dem Lokalheizelement 24 und somit in dem Bereich der Reaktionslösung 32, welche durch das Lokalheizelement 24 lokal auf die Denaturierungstemperatur erhitzt wird. Dadurch, dass nur ein sehr begrenzter Bereich der Reaktionslösung mit einem sehr geringen Volumen auf die Denaturierungstemperatur erhitzt werden muss, kann das Erhitzen und das darauffolgende Abkühlen zurück auf die Hybridisierungstemperatur in einer sehr kurzen Zeitdauer und mit einem sehr geringen Wärmeeintrag erfolgen. Durch das Erhitzen des Bereichs, in welchem die Target-Nukleinsäuren 12 angeordnet sind, auf die Denaturierungstemperatur werden diese denaturiert und lösen sich folglich von den an die magnetischen Mikropartikel 10 funktionalisierten Primern 18. Dadurch stehen die nun wieder einzelsträngig vorliegenden Target-Nukleinsäuren 12 und die wieder einzelsträngig vorliegenden (elongierten) Primer 18, die an die magnetischen Mikropartikel 10 funktionalisiert sind, für weitere Amplifikation Reaktionen bereit.

Um ein erneutes Hybridisieren und Amplifizieren der nun elongierten Primer 18 an den magnetischen Mikropartikeln 10 in der Reaktionslösung 32 zu begünstigen, werden in einem weiteren optionalen Schritt die magnetischen Mikropartikel 10 durch Bereitstellung eines entsprechenden Magnetfeldes in der Reaktionslösung 32 von dem Lokalheizelement 24 abgestoßen, sodass diese wieder in der Reaktionslösung 32 suspendiert werden und wieder frei beweglich in der Reaktionslösung 32 vorliegen. Alternativ oder zusätzlich kann optional auch eine mechanische Krafteinwirkung bereitgestellt werden, um die an das Lokalheizelement 24 angelagerten Mikropartikel 10 zumindest teilweise wieder in der Lösung zu suspendieren. Da die Reaktionslösung 32 optional und insbesondere außerhalb des erhitzten Bereichs am Lokalheizelement 24 auf der Hybridisierungstemperatur gehalten wird, wird dadurch ein erneutes Hybridisieren der Primer18 und eine darauffolgende Elongation begünstigt. Insbesondere können dabei Primer 18 mit davon verschiedenen Primern, die frei in der Reaktionslösung 32 vorliegen, hybridisieren und anschließend von einer Polymerase zu einem weiteren Doppelstrang elongiert werden, um erneut ein Amplikon der Target-Nukleinsäure 12 zu generieren. Um diesen Doppelstrang wieder aufzutrennen, kann erneut ein entsprechendes Magnetfeld bereitgestellt werden, mittels welchem die magnetischen Mikropartikel 10 zum Lokalheizelement 24 bewegt werden und dort auf die Denaturierungstemperatur erhitzt werden. Diese Schritte können beliebig oft wiederholt werden, sodass dadurch eine Polymerasekettenreaktion durchgeführt wird und eine exponentielle Amplifikation der ursprünglich in der Reaktionslösung 32 vorhandenen Target-Nukleinsäuren 12 erzielt wird. Die unterschiedlichen Magnetfelder, welche einerseits für die Anziehung der magnetischen Mikropartikel 10 an das Lokalheizelement 24 und andererseits zur Abstoßung der magnetischen Mikropartikel vom Lokalheizelement 24 erforderlich sind, können beispielsweise durch einen Permanentmagneten bereitgestellt werden, welcher in seiner Orientierung relativ zum Reaktionsgefäß 22 veränderbar ist. Alternativ oder zusätzlich kann der Magnet 28 einen oder mehrere Elektromagneten aufweisen, welche ebenfalls ein veränderbares bzw. umpolbares Magnetfeld erzeugen können.

Gemäß einer anderen optionalen Ausführungsform werden die magnetischen Mikropartikel lediglich einmal mittels eines dazu bereitgestellten Magnetfeldes zum Lokalheizelement 24 gezogen, sodass die magnetischen Mikropartikel 10 sich dort anlagern. Ein Abstoßen der Mikropartikel 10 von dem Lokalheizelement 24 erfolgt gemäß dieser Ausführungsform nicht. Vielmehr erfolgt die Amplifikation der Target-Nukleinsäure durch ein mehrmaliges lokales Aufheizen der Aufheizzone auf die Denaturierungstemperatur, wobei zwischen den Denaturierungsschritten die Temperatur in der Aufheizzone wieder auf die Annealing- bzw. Hybridisierungstemperatur abfällt. Ein Entfernen der angelagerten Mikropartikel vom Lokalheizelement ist daher für das Temperatur-Cyclen am Lokalheizelement nicht erforderlich.

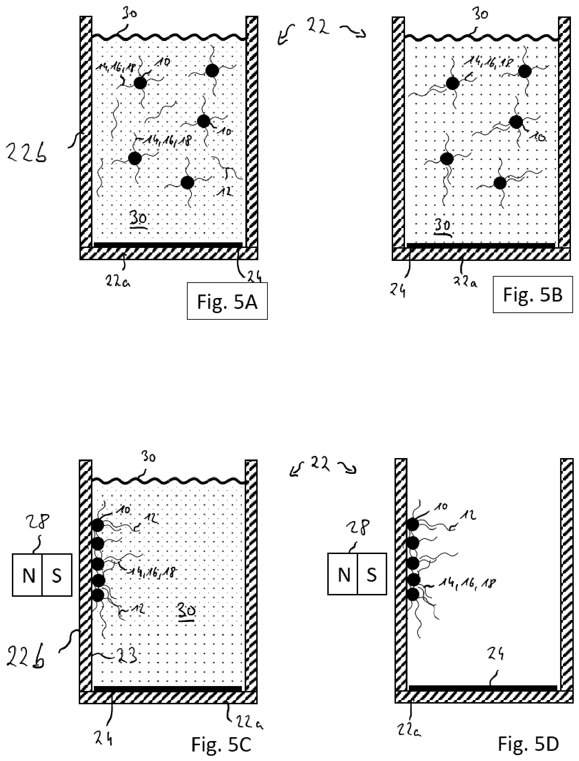

Anhand der Figuren 5A bis 5H wird im Folgenden ein Verfahren gemäß einer optionalen Ausführungsform zur Extraktion und Amplifikation einer Target-Nukleinsäure 12 erläutert.

Das Verfahren umfasst dabei sowohl eine Extraktion der Target-Nukleinsäuren 12 aus einer Probenflüssigkeit 30 sowie die Amplifikation der extrahierten Target-Nukleinsäuren 12 in einer Reaktionslösung. Gemäß der gezeigten optionalen Ausführungsform wird sowohl die Extraktion der Target-Nukleinsäuren 12 aus der Probenflüssigkeit 30 als auch die Amplifikation der Target-Nukleinsäuren 12 in der Reaktionslösung in ein und demselben Reaktionsgefäß 22 durchgeführt.

Dazu ist das Reaktionsgefäß 22 an der Innenseite dessen Boden 22a mit einem Lokalheizelement 24 ausgestattet, welches für die Extraktion nicht benötigt wird, später aber zu Amplifikation der extrahierten Target-Nukleinsäuren 12 verwendet wird. Zur besseren Übersichtlichkeit ist die Energiequelle bzw. Spannungsquelle, mittels derer das Lokalheizelement 24 beheizt werden kann, nicht dargestellt.

Die Extraktion der Target-Nukleinsäuren 12 aus der Probenflüssigkeit 30 ist in den Figuren 5A bis 5D dargestellt und entspricht im Wesentlichen dem Verfahren gemäß der optionalen Ausführungsform welche, bereits anhand der Figuren 3A bis 3D erläutert wurde. Dabei ist zu beachten, dass bei der Extraktion der Target-Nukleinsäuren 12 bzw. der magnetischen Mikropartikel 10 diese an einer Seitenwand 22b angelagert bzw. sedimentiert werden, nicht aber an dem Lokalheizelement 24, sodass die Seitenwand 22b als Extraktionselement 23 dient. Dies bietet den Vorteil, dass bei der Extraktion etwaige mitextrahierte Verunreinigungen nicht an dem Lokalheizelement 24 abgelagert werden und die spätere Amplifikationsreaktion gegebenenfalls stören bzw. beeinträchtigen. Vielmehr kann dadurch die Extraktion der magnetischen Mikropartikel 10 unter Target-Nukleinsäuren 12 zunächst von der Amplifikationsreaktion separiert werden. Auch sind gemäß diesem Verfahren die Oligonukleotide 14, die an die magnetischen Mikropartikel 10 funktionalisiert sind als Primer 18 für die darauffolgende Amplifikation Reaktion ausgebildet und haben sowohl die Funktion einer Extraktionsnukleinsäure 16 als auch die Funktion eines Primers 18 inne.

In Figur 5D liegen die extrahierten magnetischen Mikropartikel 10 und die damit verbundenen Target-Nukleinsäuren 12 angelagert an einer Seitenwand 22b des Reaktionsgefäßes 22 vor, wobei die Probenflüssigkeit 30 aus dem Reaktionsgefäß entfernt wurde und das Reaktionsgefäß 22 optional einem oder mehreren Waschschritten unterzogen wurde, um etwaige unerwünschte Rückstände aus dem Reaktionsgefäß 22 zu entfernen.

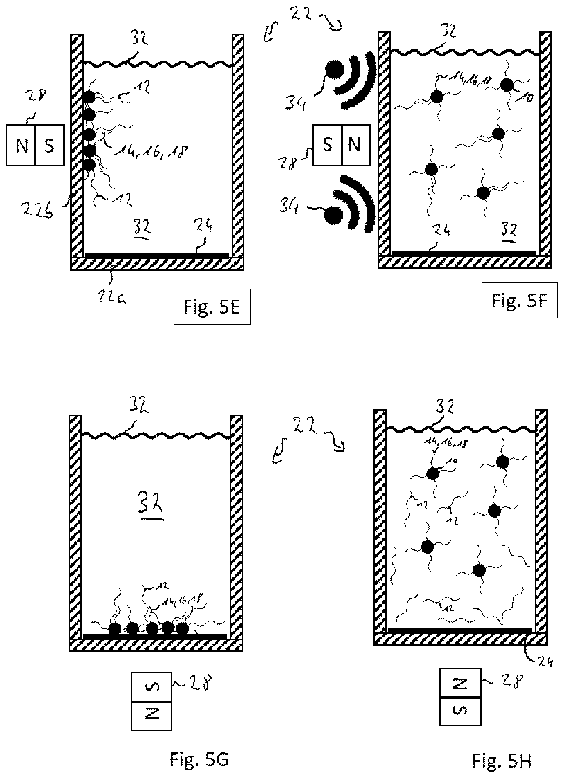

Das darauffolgende Amplifikationsverfahren umfasst eine Amplifikation der Target-Nukleinsäuren 12 in einer Reaktionslösung 32, welche anhand der Figuren 5E bis 5H erläutert wird und im Wesentlichen dem Amplifikationsverfahren entspricht, welches anhand der Figuren 4A bis 4D bereits oben erläutert wurde.

Dabei wird zunächst die Reaktionslösung 32 in das Reaktionsgefäß 22 gefüllt, wobei die Reaktionslösung 32 derart ausgebildet ist, dass diese eine Durchführung der Amplifikationsreaktion, beispielsweise einer PCR, ermöglicht (Figur 5E).

In einem weiteren Schritt, der in Figur 5F dargestellt ist, werden sodann die an die Seitenwand 22 B des Reaktionsgefäßes 22 angelagerten magnetischen Mikropartikel 10 gelöst, sodass diese zumindest teilweise in der Reaktionslösung 32 suspendiert werden. Dies kann durch ein Umpolen des Magneten 28 erfolgen, sodass das durch den Magneten 28 erzeugte Magnetfeld die magnetischen Mikropartikel 10 nicht mehr an die Seitenwand 22 bewegt, sondern von dieser abstößt. Alternativ oder zusätzlich kann eine mechanische Krafteinwirkung auf die magnetischen Mikropartikel 10 erfolgen, beispielsweise durch ein Rühren der Reaktionslösung 32 und/oder durch ein Einwirken mittels Ultraschallbeaufschlagung auf das Reaktionsgefäß 22 und/oder die Reaktionslösung 32, die in Figur 5F beispielhaft anhand der symbolisch dargestellten Ultraschallwellen 34 dargestellt. Dies führt dazu, dass die magnetischen Mikropartikel 10 mit den damit verbundenen Target-Nukleinsäuren 12 in der Reaktionslösung suspendiert sind und frei in der Reaktionslösung 32 vorliegen. Dadurch, dass in der Reaktionslösung 32 sämtliche Inhaltsstoffe vorhanden sind, die für die Durchführung der Amplifikationsreaktion erforderlich sind, wie insbesondere Enzyme und die zu den Primer 18 komplementären Primer, und die Reaktionslösung 32 in etwa auf der Hybridisierungstemperatur gehalten wird, findet eine Elongation der Primer 18 statt.

In einem weiteren Schritt, welche in Figur 5G dargestellt ist, werden die magnetischen Mikropartikel 10 in der Reaktionslösung 32 mittels eines Magnetfeldes zum Lokalheizelement 24 bewegt, sodass diese dort zumindest zum Teil sedimentieren. Dies kann beispielsweise dadurch erfolgen, dass ein Magnet 28 unterhalb des Reaktionsgefäßes 22 und insbesondere unterhalb des Lokalheizelements 24 angeordnet wird, sodass die magnetischen Mikropartikel 10 von den Magneten angezogen werden und sich entsprechend auf dem Lokalheizelement 24 ablagern. Dies führt dazu, dass die magnetischen Mikropartikel 10 und die damit verbundenen Target-Nukleinsäuren 12 in dem Bereich angeordnet sind, welche durch das Lokalheizelement 24 lokal beheizt wird, d.h. in der Aufheizzone, sodass die Denaturierungstemperatur erreicht oder überschritten wird und sich entsprechend die Target-Nukleinsäuren 12, welche an die gegebenenfalls vervollständigen Primer 18 hybridisiert sind, auftrennen und wieder freien Lösung vorliegen.

Des Weiteren werden in einem weiteren optionalen Schritt, wie in Figur 5H dargestellt, nun mittels des Magneten 28 die magnetischen Mikropartikel 10 von dem Lokalheizelement 24 abgestoßen, sodass diese wieder in der Reaktionslösung 32 suspendiert werden, und in der Reaktionslösung 32 mit Rückwärtsprimern hybridisieren können und auf diese Weise mittels eines mehrfachen Durchlaufens der in den Figuren 5G und 5H dargestellten Schritte ein exponentielle Amplifikation der Target-Nukleinsäure 12 bzw. der erzeugten Amplikons erzielen können. Dabei können optional in jedem Vervielfältigungsschritt die magnetischen Mikropartikel 10 mittels des Magneten 28 zum Lokalheizelement 24 bewegt werden, dort mittels des Lokalheizelement 24 durch lokales Heizen auf die Denaturierungstemperatur erhitzt werden und nach der Denaturierung durch ein umpolen des Magneten 28 wieder von dem Lokalheizelement 24 entfernt werden, um in der auf die Hybridisierungstemperatur temperierten Reaktionslösung 32 wieder mit Rückwärtsprimern und/oder Target-Nukleinsäuren 12 zu hybridisieren.

Beispielsweise kann eine Detektion der erzeugten Amplikons mit optischen Mitteln erfolgen. Dazu können beispielsweise solche Primer eingesetzt werden, welche mit einem Farbstoff und einem Quencher ausgebildet sind und nur dann ein Fluoreszenzsignal bereitstellen, wenn diese durch eine Polymerase Teil eines Amplikons geworden sind und dadurch der Farbstoff von dem Quencher getrennt wurde. Zur optischen Detektion kann es vorteilhaft sein, wenn das Reaktionsgefäß 22 zumindest teilweise transparent für die Fluoreszenzwellenlänge des Farbstoffs und eine dafür vorgesehene Anregungswellenlänge ausgestaltet ist. Beispielsweise können die Wände 22b, der Boden 22a und/oder der Deckel 22c des Reaktionsgefäßes 22 transparent ausgebildet sein. Auch kann eine Messung von oben durch den Deckel 22c vertikal nach unten durch die Reaktionslösung 32 und durch das Lokalheizelement 24 den Boden 22a erfolgen. Dazu kann es vorteilhaft sein, wenn das Lokalheizelement 24 eine oder mehrere Ausnehmungen aufweist, durch welche das Licht zur Detektion der Amplikons zumindest teilweise hindurchtreten kann, um unterhalb des Reaktionsgefäß ist 22 detektiert zu werden.

Geeignete Parameter für die Durchführung einer Amplifikationsreaktion und insbesondere einer PCR, insbesondere hinsichtlich geeigneter Inhaltsstoffe der Reaktionslösung, können beispielsweise der Druckschrift DE102016120124A1 entnommen werden. Auch können der DE102016120124A1 beispielhafte Angaben hinsichtlich der Temperaturen und Zeitdauern für das lokale Heizen entnommen werden, sodass diesbezüglich auf die bereits veröffentlichte Druckschrift verwiesen wird.

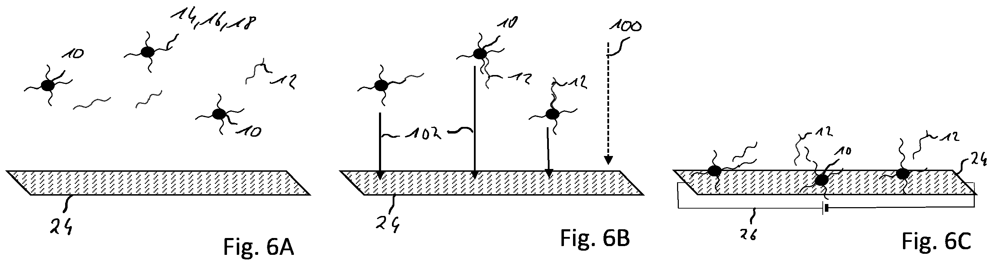

Anhand der Figuren 6A bis 6C wird nochmals das Prinzip einer Amplifikation gemäß einer optionalen Ausführungsform schematisch erläutert. Dabei sind die Darstellungen im Wesentlichen auf das Lokalheizelement 24, die magnetischen Mikropartikel 10 und die Target-Nukleinsäuren 12 reduziert.

In Figur 6A sind die magnetischen Mikropartikel 10 und die Target-Nukleinsäuren 12 in der Reaktionslösung (nicht gezeigt) suspendiert und frei beweglich, wobei derartige Bedingungen vorherrschen, dass eine Hybridisierung der Target-Nukleinsäuren 12 an die Oligonukleotide 14, mit denen die magnetischen Mikropartikel funktionalisiert sind, ermöglicht wird und stattfindet.

In Figur 6B ist ein Schritt gezeigt, in welchem ein Magnetfeld bereitgestellt wird, welches durch den Pfeil 100 symbolisch dargestellt ist. Das Magnetfeld 100 übt eine Kraft 102 auf die magnetischen Mikropartikel 10 aus, sodass diese sich die Richtung des Pfeils 102 zum Lokalheizelement 24 bewegen und sich dort anlagern.

In Figur 6C ist eine weiterer Schritt gezeigt, in welchem die magnetischen Mikropartikel 10 an dem Lokalheizelement 24 angelagert sind und mittels des Lokalheizelements 24 der Bereich der Reaktionslösung lokal erhitzt wird, in welchem sich die angelagerten Mikropartikel 10 befinden, sodass in dem Bereich die Denaturierungstemperatur erreicht oder überschritten wird und sich die an die Oligonukleotide 14 hybridisierten Target-Nukleinsäuren 12 von den Oligonukleotiden 14 trennen.

Anschließend können die Mikropartikel 10 ggf. von dem Lokalheizelement abgestoßen werden, beispielsweise durch ein Magnetfeld, welches nun in die entgegengesetzte Richtung wirkt, sodass die Mikropartikel 10 wieder in der Reaktionslösung suspendiert werden und sich dort verteilen, um erneut für eine Hybridisierung zur Verfügung zu stehen.

Bezugszeichenliste

| | |
|---|---|
| 10 | magnetisches Mikropartikel |
| 10a | Beschichtung des Mikropartikels |
| 12 | Target-Nukleinsäure |
| 14 | Oligonukleotid |
| 16 | Extraktionsnukleinsäure |
| 18 | Primer |
| 20 | Vorrichtung zur Amplifikation einer Target-Nukleinsäure |
| 22 | Reaktionsgefäß |
| 22a | Boden des Reaktionsgefäßes |
| 22b | Wand des Reaktionsgefäßes |
| 22c | Deckel des Reaktionsgefäßes |
| 23 | Extraktionselement |
| 24 | Lokalheizelement |
| 26 | Spannungsquelle |
| 28 | Magnet |
| 30 | Probenflüssigkeit |
| 32 | Reaktionslösung |
| 34 | Ultraschallwellen |

**Patentansprüche**

1. Verfahren zur Amplifikation einer Target-Nukleinsäure (12), das Verfahren umfassend die Schritte:

    a) Bereitstellen einer die Target-Nukleinsäure (12) beinhaltenden Probenflüssigkeit (30) in einem Reaktionsgefäß (22) und zumindest eines Lokalheizelementes (24) in direktem Kontakt mit der Probenflüssigkeit;
    b) Bereitstellen magnetischer Mikropartikel (10) in der Probenflüssigkeit (30), wobei die magnetischen Mikropartikel (10) jeweils mit zumindest einem Primer (18) für die Amplifikation der Target-Nukleinsäure (12) funktionalisiert sind;
    c) Hybridisieren der Target-Nukleinsäure (12) mit zumindest einem der auf den magnetischen Mikropartikeln (10) funktionalisierten Primern (18);
    d) Bereitstellen eines Magnetfelds im Reaktionsgefäß (22) derart, dass sich zumindest ein Teil der magnetischen Mikropartikel (10) mit der daran hybridisierten Target-Nukleinsäure (12) an das Lokalheizelement (24) anlagert;
    e) Entfernen der Probenflüssigkeit (30) aus dem Reaktionsgefäß (22);
    f) Durchführen eines oder mehrerer Waschschritte, denen das Reaktionsgefäß unterzogen wird;
    g) Bereitstellen einer Reaktionslösung (32) zur Durchführung einer Amplifikationsreaktion der Target-Nukleinsäure (12) in dem Reaktionsgefäß (22);
    h) Lokales Erhitzen der Reaktionslösung (32) auf eine Denaturierungstemperatur mittels des Lokalheizelements (24) in dem Bereich, in welchem die magnetischen Mikropartikel (10) an das Lokalheizelement (24) angelagert sind, wobei eine Heizdauer des Lokalheizelements (24) zum lokalen Erhitzen der Reaktionslösung (32) auf die Denaturierungstemperatur nicht mehr als 20 ms pro Denaturierungsschritt beträgt.

2. Verfahren gemäß Anspruch 1, wobei das Lokalheizelement (24) eine oder mehrere elektrisch beheizbare Metallfolien umfasst.

3. Verfahren gemäß Anspruch 1 oder 2, wobei der mittels des Lokalheizelements (24) erhitzte bzw. beheizte Bereich der Reaktionslösung (32) einen sich vom Lokalheizelement (24) während des Erhitzens weg erstreckenden Temperaturgradienten aufweist und wobei sich der Betrag des Temperaturgradienten optional auf einer Länge zwischen 1 $\mu$m und 10 $\mu$m von der Oberfläche des Lokalheizelements halbiert.

4. Verfahren gemäß einem der vorhergehenden Ansprüche, wobei beim lokalen Erhitzen des Bereichs der Reaktionslösung, in welchem die magnetischen Mikropartikel (10) angelagert sind, die Reaktionslösung außerhalb des lokal erhitzten Bereichs im Wesentlichen isothermal bleibt.

5. Verfahren gemäß einem der vorhergehenden Ansprüche, wobei das lokale Erhitzen der Reaktionslösung (32) auf die Denaturierungstemperatur mittels des Lokalheizelements (24) derart erfolgt, dass eine Wärmediffusionsweite in die Reaktionslösung (32) senkrecht zur Oberfläche des Lokalheizelements (24) in einem Bereich von 0,05 µm bis 200 µm liegt.

6. Verfahren gemäß einem der vorhergehenden Ansprüche, wobei das Lokalheizelement (24) zumindest teilweise aus ferromagnetischem Material ausgebildet ist.

7. Verfahren gemäß Anspruch 6, wobei das Lokalheizelement (24) eine Folie und/oder einen Draht aufweist, oder als Folie oder Draht ausgebildet ist.

8. Verfahren gemäß Anspruch 7, wobei das Lokalheizelement (24) an einer Gefäßwand des Reaktionsgefäßes (22) ausgebildet ist und/oder einen Teil einer Gefäßwand (22) des Reaktionsgefäßes bildet.

9. Verfahren nach einem der vorhergehenden Ansprüche, wobei das Magnetfeld durch einen Magneten (28) bereitgestellt wird und wobei der Magnet (28) auf einer dem Reaktionsgefäß (22) abgewandten Seite des Lokalheizelements (24) ausgebildet ist.

10. Verfahren gemäß Anspruch 9, wobei der Magnet (28) einen in seiner Position und/oder Orientierung relativ zum Reaktionsgefäß (22) veränderbaren Permanentmagneten und/oder einen Elektromagneten aufweist.

11. Vorrichtung (20) zur Amplifikation einer Target-Nukleinsäure (12), die Vorrichtung (20) umfassend:

- Ein Reaktionsgefäß (22), welches dazu ausgelegt ist, eine die Target-Nukleinsäure (12) enthaltende Reaktionslösung (32) aufzunehmen:
- ein Lokalheizelement (24), welches derart in und/oder an dem Reaktionsgefäß (22) angeordnet ist, dass sich das Lokalheizelement (24) bei mit Reaktionslösung (32) befülltem Reaktionsgefäß (22) zumindest teilweise in direktem Kontakt mit der Reaktionslösung (32) befindet, und welches dazu angepasst ist, die Reaktionslösung (32) in einer Heizdauer von nicht mehr als 20 ms pro Denaturierungsschritt lokal auf die Denaturierungstemperatur zu erhitzen;

- einen Magneten (28) zur Erzeugung eines Magnetfeldes, wobei das Magnetfeld auf zumindest einen Teil der in der Reaktionslösung (32) befindliche magnetische Mikropartikel (10) derart wirkt, dass diese sich an dem Lokalheizelement (24) anlagern, sodass der zumindest eine Teil der magnetischen Mikropartikel (10) am Lokalheizelement (24) durch das bereitgestellte Magnetfeld sedimentiert.

12. Vorrichtung gemäß Anspruch 11, wobei das Lokalheizelement (24) zumindest teilweise aus ferromagnetischem Material ausgebildet ist.

13. Vorrichtung (20) gemäß Anspruch 11 oder 12, wobei das Lokalheizelement (24) zumindest einen Teil einer Gefäßwand (22a, 22b, 22c) des Reaktionsgefäßes (22) bildet.

14. Vorrichtung (20) gemäß einem der Ansprüche 11 bis 13, wobei der Magnet (28) einen in seiner Position und/oder Orientierung relativ zum Reaktionsgefäß (22) veränderbaren Permanentmagneten und/oder einen Elektromagneten aufweist.

**Claims**

1. Method for amplifying a target nucleic acid (12), the method comprising the following steps:

a) providing a sample fluid (30) containing the target nucleic acid (12) in a reaction container (22) and at least one local heating element (24) in direct contact with the sample fluid;
b) providing magnetic microparticles (10) in the sample fluid (30), wherein the magnetic microparticles (10) are each functionalized with at least one primer (18) for amplifying the target nucleic acid (12);
c) hybridizing the target nucleic acid (12) with at least one of the primers (18) functionalized on the magnetic microparticles (10);
d) providing a magnetic field in the reaction container (22) such that at least some of the magnetic microparticles (10) with the target nucleic acid (12) hybridized thereto attach to the local heating element (24);
e) removing the sample fluid (30) from the reaction container (22);
f) performing one or more washing steps to which the reaction container is subjected;
g) providing a reaction solution (32) for performing an amplification reaction of the target nucleic acid (12) in the reaction container (22);
h) locally heating the reaction solution (32) to a denaturation temperature by means of the local heating element (24) in the region in which the magnetic microparticles (10) are attached to the local heating element (24), wherein a heating period of the local heating element (24) for locally heating the reaction solution (32) to the denaturation temperature is not more than 20 ms per denaturation step.

2. Method according to claim 1, wherein the local heating element (24) comprises one or more electrically heatable metal foils.

3. Method according to either claim 1 or claim 2, wherein the region of the reaction solution (32) heated by means of the local heating element (24) has a temperature gradient extending away from the local heating element (24) during heating, and wherein the magnitude of the temperature gradient optionally halves along a length between 1 $\mu$m and 10 $\mu$m from the surface of the local heating element.

4. Method according to any of the preceding claims, wherein upon local heating of the region of the reaction solution in which the magnetic microparticles (10) are attached, the reaction solution outside the locally heated region remains substantially isothermal.

5. Method according to any of the preceding claims, wherein local heating of the reaction solution (32) to the denaturation temperature by means of the local heating element (24) is performed in such a manner that a heat diffusion distance into the reaction solution (32) perpendicularly to the surface of the local heating element (24) is in a range of 0.05 $\mu$m to 200 $\mu$m.

6. Method according to any of the preceding claims, wherein the local heating element (24) is at least partly formed of ferromagnetic material.

7. Method according to claim 6, wherein the local heating element (24) comprises a foil and/or a wire, or is formed as a foil or a wire.

8. Method according to claim 7, wherein the local heating element (24) is formed on a container wall of the reaction container (22) and/or forms a part of a container wall (22) of the reaction container.

9. Method according to any of the preceding claims, wherein the magnetic field is provided by a magnet (28) and wherein the magnet (28) is formed on a side of the local heating element (24) facing away from the reaction container (22).

10. Method according to claim 9, wherein the magnet (28) has a permanent magnet and/or an electromagnet which is variable in position and/or orientation relative to the reaction container (22).

11. Device (20) for amplifying a target nucleic acid (12), the device (20) comprising:

- a reaction container (22) which is designed to receive a reaction solution (32) containing the target nucleic acid (12):
- a local heating element (24) which is arranged in and/or on the reaction container (22) such that the local heating element (24) is at least partly in direct contact with the reaction solution (32) when the reaction container (22) is filled with the reaction solution (32), and which is adapted to locally heat the reaction solution (32) to the denaturation temperature in a heating period of not more than 20 ms per denaturation step;
- a magnet (28) for generating a magnetic field, wherein the magnetic field acts on at least some of the magnetic microparticles (10) in the reaction solution (32) in such a way that they attach to the local heating element (24), so that at least some of the magnetic microparticles (10) sediment on the local heating element (24) due to the provided magnetic field.

12. Device according to claim 11, wherein the local heating element (24) is at least partly formed of ferromagnetic material.

13. Device (20) according to either claim 11 or claim 12, wherein the local heating element (24) forms at least a part of a container wall (22a, 22b, 22c) of the reaction container (22).

14. Device (20) according to any of claims 11 to 13, wherein the magnet (28) has a permanent magnet and/or an electromagnet which is variable in position and/or orientation relative to the reaction container (22).

**Revendications**

1. Procédé destiné à l'amplification d'un acide nucléique cible (12), le procédé comprenant les étapes consistant à :

a) mettre à disposition un liquide échantillon (30) contenant l'acide nucléique cible (12) dans un récipient de réaction (22) et au moins un élément de chauffage local (24) en contact direct avec le liquide échantillon ;
b) mettre à disposition des microparticules magnétiques (10) dans le liquide échantillon (30), dans lequel les microparticules magnétiques (10) sont respectivement fonctionnalisées avec au moins une amorce (18) pour l'amplification de l'acide nucléique cible (12) ;

c) hybrider l'acide nucléique cible (12) avec au moins l'une des amorces (18) fonctionnalisées sur les microparticules magnétiques (10) ;

d) mettre à disposition un champ magnétique dans le récipient de réaction (22) de telle sorte qu'au moins une partie des microparticules magnétiques (10) comportant l'acide nucléique cible (12) hybridé sur celles-ci se fixe sur l'élément de chauffage local (24) ;

e) éliminer le liquide échantillon (30) du récipient de réaction (22) ;

f) réaliser une ou plusieurs étapes de lavage auxquelles le récipient de réaction est soumis ;

g) mettre à disposition une solution réactionnelle (32) pour la réalisation d'une réaction d'amplification de l'acide nucléique cible (12) dans le récipient de réaction (22) ;

h) chauffer localement la solution réactionnelle (32) à une température de dénaturation au moyen de l'élément de chauffage local (24) dans la zone où les microparticules magnétiques (10) sont fixées sur l'élément de chauffage local (24), dans lequel une durée de chauffage de l'élément de chauffage local (24) pour chauffer localement la solution réactionnelle (32) à la température de dénaturation n'est pas supérieure à 20 ms par étape de dénaturation.

2. Procédé selon la revendication 1, dans lequel l'élément de chauffage local (24) comprend une ou plusieurs feuilles métalliques pouvant être chauffées électriquement.

3. Procédé selon la revendication 1 ou 2, dans lequel la zone de la solution réactionnelle (32) chauffée au moyen de l'élément de chauffage local (24) présente un gradient de température s'éloignant de l'élément de chauffage local (24) pendant le chauffage, et dans lequel la valeur du gradient de température est éventuellement divisée par deux sur une longueur comprise entre 1 $\mu$m et 10 $\mu$m à partir de la surface de l'élément de chauffage local.

4. Procédé selon l'une des revendications précédentes, dans lequel, lors du chauffage local de la zone de la solution réactionnelle où les microparticules magnétiques (10) sont fixées, la solution réactionnelle reste sensiblement isotherme en dehors de la zone chauffée localement.

5. Procédé selon l'une des revendications précédentes, dans lequel le chauffage local de la solution réactionnelle (32) à la température de dénaturation au moyen de l'élément de chauffage local (24) est effectué de telle sorte qu'une distance de diffusion de chaleur dans la solution réactionnelle (32) perpendiculairement à la surface de l'élément de chauffage local (24) se situe dans une plage allant de 0,05 $\mu$m à 200 $\mu$m.

6. Procédé selon l'une des revendications précédentes, dans lequel l'élément de chauffage local (24) est réalisé au moins en partie à partir d'un matériau ferromagnétique.

7. Procédé selon la revendication 6, dans lequel l'élément de chauffage local (24) présente une feuille et/ou un fil, ou est réalisé comme une feuille ou un fil.

8. Procédé selon la revendication 7, dans lequel l'élément de chauffage local (24) est réalisé sur une paroi de récipient du récipient de réaction (22) et/ou forme une partie d'une paroi de récipient (22) du récipient de réaction.

9. Procédé selon l'une des revendications précédentes, dans lequel le champ magnétique est mis à disposition par un aimant (28) et dans lequel l'aimant (28) est réalisé sur un côté de l'élément de chauffage local (24) opposé au récipient de réaction (22).

10. Procédé selon la revendication 9, dans lequel l'aimant (28) présente un aimant permanent et/ou un électroaimant dont la position et/ou l'orientation par rapport au récipient de réaction (22) peuvent être modifiées.

11. Dispositif (20) pour l'amplification d'un acide nucléique cible (12), le dispositif (20) comprenant :

- un récipient de réaction (22) qui est conçu pour recevoir une solution réactionnelle (32) contenant l'acide nucléique cible (12) :
- un élément de chauffage local (24) qui est disposé dans le récipient de réaction (22) et/ou au niveau de celui-ci de telle sorte que l'élément de chauffage local (24) se trouve au moins partiellement en contact direct avec la solution réactionnelle (32) lorsque le récipient de réaction (22) est rempli avec la solution réactionnelle (32), et qui est adapté pour chauffer la solution réactionnelle (32) localement à la température de dénaturation pendant une durée de chauffage ne dépassant pas 20 ms par étape de dénaturation ;
- un aimant (28) pour la génération d'un champ magnétique, dans lequel le champ magnétique agit sur au moins une partie des microparticules magnétiques (10) se trouvant dans la solution réactionnelle (32) de telle sorte que celles-ci se fixent sur l'élément de chauffage local (24), de sorte que l'au moins une partie des microparticules magnétiques (10) se sédimente sur l'élément de chauffage local (24) par le champ magnétique mis à disposition.

**12.** Dispositif selon la revendication 11, dans lequel l'élément de chauffage local (24) est réalisé au moins en partie à partir d'un matériau ferromagnétique.

**13.** Dispositif (20) selon la revendication 11 ou 12, dans lequel l'élément de chauffage local (24) forme au moins une partie d'une paroi de récipient (22a, 22b, 22c) du récipient de réaction (22).

**14.** Dispositif (20) selon l'une des revendications 11 à 13, dans lequel l'aimant (28) présente un aimant permanent et/ou un électroaimant dont la position et/ou l'orientation par rapport au récipient de réaction (22) peuvent être modifiées.

Fig. 1

Fig. 2

Fig. 3A

Fig. 3B

Fig. 3C

Fig. 3D

Fig. 4A

Fig. 4B

Fig. 4C

Fig. 4D

Fig. 5A

Fig. 5B

Fig. 5C

Fig. 5D

Fig. 5E

Fig. 5F

Fig. 5G

Fig. 5H

Fig. 6A

Fig. 6B

Fig. 6C

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 4409436 A1
- WO 2008020381 A2
- WO 2015086652 A1
- WO 2012006116 A2
- WO 8911546 A1
- DE 102016120124 A1

**In der Beschreibung aufgeführte Nicht-Patentliteratur**

- **D. RENNEBERG** ; **C.J. LEUMANN**. Watson-Crick base-pairing properties of Tricyclo-DNA. *J. Am. Chem. Soc.*, 2002, vol. 124, 5993-6002